(19) Europäisches Patentamt
European Patent Office
Office européen des brevets

(11) EP 3 992 981 A1

(12) EUROPEAN PATENT APPLICATION

(43) Date of publication:
04.05.2022 Bulletin 2022/18

(21) Application number: 21205526.3

(22) Date of filing: 29.10.2021

(51) International Patent Classification (IPC):
G16H 30/40 (2018.01)     G16H 20/40 (2018.01)
G16H 50/50 (2018.01)     G16H 50/20 (2018.01)

(52) Cooperative Patent Classification (CPC):
G16H 30/40; G16H 20/40; G16H 50/20;
G16H 50/50

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 30.10.2020 JP 2020183315

(71) Applicant: Canon Medical Systems Corporation
Otawara-shi, Tochigi 324-0036 (JP)

(72) Inventors:
• Ooida, Junichi
Tokyo (JP)

• Ueyama, Kenichi
Tokyo (JP)
• Oguchi, Yuichiro
Tokyo (JP)
• Kiyohara, Naoki
Tokyo (JP)
• Aoyama, Gakuto
Tochigi (JP)

(74) Representative: Moreland, David
Marks & Clerk LLP
Aurora
120 Bothwell Street
Glasgow G2 7JS (GB)

(54) **MEDICAL DATA PROCESSING DEVICE, AND MEDICAL DATA PROCESSING METHOD**

(57) A medical data processing device according to an embodiment includes a first acquisition unit (155a), a calculation unit (155b), and an estimation unit (155d). The first acquisition unit (155a) acquires estimated data related to a state of a biological organ at a first timing, and measured data indicating the state of the biological organ at the first timing. The calculation unit (155b) calculates a parameter based on the estimated data and the measured data. The estimation unit (155d) estimates a state of the biological organ in a predetermined time phase at a second timing different from the first timing based on the parameter and the estimated data in the predetermined time phase.

FIG.2

```
                START
                  │
                  ▼  S1
        ACQUIRE MEDICAL IMAGE
                  │
                  ▼  S2
       ACQUIRE FORM INFORMATION
                  │
                  ▼  S3
           SET PARAMETER
                  │
                  ▼  S4
        PERFORM PRETREATMENT
             SIMULATION
                  │
                  ▼  S5
      ┌─────────────────────┐
 NO   │  IS RESULT OF       │
◄─────│ PRETREATMENT SIMULATION│
      │   DETERMINED?       │
      └─────────────────────┘
                  │ YES
                  ▼  S6
       POSTTREATMENT SIMULATION
                  │
                  ▼  S7
       DISPLAY ESTIMATION RESULT
                  │
                  ▼
                 END
```

EP 3 992 981 A1

**Description**

FIELD

Embodiments described herein relate generally to a medical data processing device, and a medical data processing method.

BACKGROUND

At the time of planning a treatment policy for a subject (patient) having a heart disease, a doctor determines the treatment policy based on the doctor's knowledge and experiences, using a state of the subject and a form or fluid information of a heart to be treated, which are obtained before treatment. Ideally, the doctor should plan the treatment policy so that the state of the subject becomes optimum after the treatment. For that, required is a technique of estimating the state of the subject after the treatment from information about the state of the subject before the treatment.

However, it is difficult to establish a technique of estimating the state after the treatment from the state before the treatment. One of the factors is that a shape, movement, a blood flow, and the like of a heart are greatly different among individuals.

Summary of Invention

A medical data processing device provided according to one aspect of the present invention includes a first acquisition unit, a calculation unit, and an estimation unit. The first acquisition unit acquires estimated data related to a state of a biological organ at a first timing, and measured data indicating the state of the biological organ at the first timing. The calculation unit calculates a parameter based on the estimated data and the measured data. The estimation unit estimates a state of the biological organ in a predetermined time phase at a second timing different from the first timing based on the parameter and the estimated data in the predetermined time phase.

The calculation unit may calculate, as the parameter, a parameter related to a shape of a first biological organ based on the estimated data and the measured data, and the estimation unit may estimate the state of the biological organ in the predetermined time phase at the second timing based on the parameter related to the shape of the first biological organ and the estimated data in the predetermined time phase.

The calculation unit may calculate, as the parameter related to the shape, a parameter indicating at least one of hardness, a thickness, a fiber direction, a length, a width, a connecting position, and the number of the first biological organ based on the estimated data and the measured data, and the estimation unit may estimate the state of the biological organ in the predetermined time phase at the second timing based on the parameter indicating at least one of the above items and the estimated data in the predetermined time phase.

A medical data processing device provided according to another aspect of the present invention includes a first acquisition unit, a calculation unit, a second acquisition unit, and the estimation unit. The first acquisition unit acquires estimated data related to a state of a biological organ at a first timing, and measured data indicating the state of the biological organ at the first timing. The calculation unit calculates a parameter based on the estimated data and the measured data. The second acquisition unit acquires state data related to a state of the biological organ in a predetermined time phase at a second timing different from the first timing. The estimation unit estimates a state of the biological organ at the second timing over a plurality of time phases based on the state data and the parameter.

The calculation unit may calculate, as the parameter, a parameter related to movement of a second biological organ based on the estimated data and the measured data, and the estimation unit may estimate the state of the biological organ at the second timing over the time phases based on the state data and the parameter related to movement of the second biological organ.

The calculation unit may calculate, as the parameter related to movement, a parameter indicating at least one of hardness, a volume, and a degree of smoothness of a surface of the second biological organ, and viscosity, a flow speed, and a total quantity of a fluid flowing in the second biological organ based on the estimated data and the measured data, and the estimation unit may estimate the state of the biological organ at the second timing over the time phases based on the state data and the parameter indicating at least one of the above items.

The calculation unit may calculate the parameter used for estimating the estimated data in a case in which an evaluation value that is calculated based on the estimated data and the measured data satisfies a standard determined in advance.

The calculation unit may calculate the parameter used for estimating the estimated data in a case in which the estimated data is similar to the measured data.

The calculation unit may calculate the parameter used for estimating the estimated data in a case in which a difference between the estimated data and the measured data is equal to or smaller than a threshold.

The calculation unit may calculate the evaluation value based on a point and a surface constituting the estimated data, and a point and a surface constituting the measured data.

The calculation unit may repeatedly change the parameter used for estimating the estimated data until the evaluation value satisfies the standard.

The calculation unit may repeatedly change the parameter used for estimating the estimated data until the estimated data becomes similar to the measured data.

The calculation unit may repeatedly change the parameter used for estimating the estimated data until the difference becomes equal to or smaller than the threshold.

The first acquisition unit may acquire a plurality of pieces of the estimated data by estimating the pieces of estimated data using a plurality of different parameters, and the calculation unit may calculate the parameter used for estimating the state of the biological organ based on the pieces of estimated data and the measured data.

The calculation unit may preferentially change a parameter with which the evaluation value comes closer to a reference value as compared with the other parameters among the parameters used for estimating the estimated data, and the first acquisition unit may estimate, every time the calculation unit preferentially changes the parameter with which the evaluation value comes closer to the reference value than the other parameters, the estimated data using the changed parameter.

The first acquisition unit may acquire the estimated data at a timing before the treatment as the first timing, and the measured data at the timing before the treatment. The estimation unit may estimate the state of the biological organ at a timing after the treatment as the second timing.

The first acquisition unit may acquire the estimated data related to a state of a mitral valve as the biological organ, and the measured data indicating the state of the mitral valve. The estimation unit may estimate the state of the mitral valve at the second timing.

The first acquisition unit may acquire the estimated data related to a state of a form of the biological organ as the state of the biological organ, and the measured data indicating the state of the form of the biological organ. The estimation unit may estimate the state of the form of the biological organ at the second timing.

The first acquisition unit may acquire the estimated data related to a state of a blood flow in the biological organ as the state of the biological organ, and the measured data indicating the state of the blood flow in the biological organ. The estimation unit may estimate the state of the blood flow in the biological organ at the second timing.

The first acquisition unit may acquire the estimated data and the measured data by using medical images of at least two time phases among medical images of three or more time phases at the first timing in which the biological organ is delineated.

The calculation unit may further change a calculation parameter for performing processing of estimating the estimated data, and the first acquisition unit may further estimate the estimated data based on a changed calculation parameter.

The medical data processing device may further include a display control unit that controls a display unit to display a plurality of parameters used for estimating the respective pieces of estimated data, and the respective evaluation values based on the pieces of estimated data and the measured data.

The estimation unit may estimate state data related to a state of a fluid at the second timing based on the estimated state of the form of the biological organ and an electric circuitry model related to hemodynamics of a living body set in advance.

The estimation unit may estimate the state of the biological organ in the predetermined time phases at the second timing based on the parameter and the pieces of estimated data in the predetermined time phases.

A medical data processing device provided according to yet another aspect of the present invention includes an acquisition unit and an estimation unit. The acquisition unit acquires state data related to a state of a form of a biological organ at a specific timing. The estimation unit estimates, based on both of the state data and an electric circuitry model related to hemodynamics of a living body set in advance, state data related to a state of a fluid at the specific timing.

The estimation unit may estimate state data indicating a state of a backward flow amount of a blood flow as the state data related to the state of the fluid at the specific timing based on the state data and the electric circuitry model.

A medical data processing device provided according to yet another aspect of the present invention includes a first acquisition unit, a calculation unit, and an estimation unit. The first acquisition unit acquires estimated data related to a state of a coronary artery at a first timing, and measured data indicating the state of the coronary artery at the first timing. The calculation unit calculates a parameter based on the estimated data and the measured data. The estimation unit estimates a state of the coronary artery at a second timing different from the first timing based on the parameter.

The first acquisition unit may acquire the estimated data related to a state of a form of the coronary artery, and the measured data indicating the state of the form of the coronary artery. The estimation unit may estimate a wall shearing stress on the coronary artery at the second timing.

The first acquisition unit may acquire the estimated data related to a state of a heart as the biological organ, and the measured data indicating the state of the heart. The second acquisition unit may acquire the state data related to the state of the heart in the predetermined time phase. The estimation unit may estimate a state of the heart at the second timing over a plurality of time phases.

The first acquisition unit may acquire the estimated data related to the state of the form of the heart as the state of the heart, and the measured data indicating the state of the form of the heart. The second acquisition unit may acquire the state data related to the state of the form of the heart. The estimation unit may estimate a state of the form of the heart at the second timing or an electric path of a stimulus conduction system of the heart over a plurality of time phases.

The first acquisition unit may acquire the estimated data related to a state of lungs as the biological organ, and the measured data indicating the state of the lungs. The second acquisition unit may acquire the state data related to the state of the lungs in the predetermined time phase. The estimation unit may estimate a state of the lungs at the second timing over a plurality of time phases.

The first acquisition unit may acquire the estimated data related to a state of a form of the lungs as the state of the lungs, and the measured data indicating the state of the form of the lungs. The second acquisition unit may acquire the state data related to the state of the form of the lungs. The estimation unit may estimate a respiratory volume of the lungs at the second timing over a plurality of time phases.

The first acquisition unit may acquire the estimated data by performing a simulation for acquiring the estimated data, and the calculation unit may calculate, as the parameter, a parameter for bringing the estimated data closer to the measured data and for adjusting the simulation.

The estimation unit may estimate a state of a valve over at least one cycle of time phase with respect to movement of the valve.

A medical data processing method provided according to one aspect of the present invention includes: acquiring estimated data related to a state of a biological organ at a first timing, and measured data indicating the state of the biological organ at the first timing; calculating a parameter based on the estimated data and the measured data; and estimating a state of the biological organ in a predetermined time phase at a second timing different from the first timing based on the parameter and the estimated data in the predetermined time phase.

A medical data processing method provided according to another aspect of the present invention includes: acquiring estimated data related to a state of a biological organ at a first timing, and measured data indicating the state of the biological organ at the first timing; calculating a parameter based on the estimated data and the measured data; acquiring state data related to a state of the biological organ in a predetermined time phase at a second timing different from the first timing; and estimating a state of the biological organ at the second timing over a plurality of time phases based on the state data and the parameter.

A medical data processing method provided according to yet another aspect of the present invention includes: acquiring state data related to a state of a form of a biological organ at a specific timing; and estimating state data related to a state of a fluid at the specific timing based on both of the state data and an electric circuitry model related to hemodynamics of a living body set in advance.

A medical data processing program provided according to one aspect of the present invention is a computer program for causing a computer to perform processing of: acquiring estimated data related to a state of a biological organ at a first timing, and measured data indicating the state of the biological organ at the first timing; calculating a parameter based on the estimated data and the measured data; and estimating a state of the biological organ in a predetermined time phase at a second timing different from the first timing based on the parameter and the estimated data in the predetermined time phase.

A medical data processing program provided according to another aspect of the present invention is a computer program for causing a computer to perform processing of: acquiring estimated data related to a state of a biological organ at a first timing, and measured data indicating the state of the biological organ at the first timing; calculating a parameter based on the estimated data and the measured data; acquiring state data related to a state of the biological organ in a predetermined time phase at a second timing different from the first timing; and estimating a state of the biological organ at the second timing over a plurality of time phases based on the state data and the parameter.

A medical data processing program provided according to yet another aspect of the present invention is a computer program for causing a computer to perform processing of: acquiring state data related to a state of a form of a biological organ at a specific timing; and estimating state data related to a state of a fluid at the specific timing based on both of the state data and an electric circuitry model related to hemodynamics of a living body set in advance.

BRIEF DESCRIPTION OF THE DRAWINGS

FIG. 1 is a diagram illustrating a configuration example of a medical data processing system and a medical data processing device according to a first embodiment;

FIG. 2 is a flowchart illustrating a processing procedure of processing performed by processing functions included in processing circuitry of the medical data processing device according to the first embodiment;

FIG. 3 is a diagram for explaining an example of processing at Step S2 according to the first embodiment;

FIG. 4 is a diagram illustrating an example of a user interface according to the first embodiment;

FIG. 5 is a diagram for explaining an example of processing at Step S4 according to the first embodiment;

FIG. 6 is a diagram for explaining an example of processing performed by a second acquisition function according to the first embodiment;

FIG. 7 is a diagram for explaining an example of processing performed by an estimation function according to the first embodiment;

FIG. 8 is a diagram illustrating an example of a table according to a fourth modification;

FIG. 9 is a diagram for explaining an example of processing at Step S6 according to the first embodiment;

FIG. 10 is a diagram for explaining an example of processing at Step S6 according to a fifth modification; and

FIG. 11 is a diagram for explaining an example of processing performed by an estimation function according to a sixth modification.

DETAILED DESCRIPTION

One of problems to be solved by embodiments disclosed in the specification and the drawings is to more appropriately estimate a state of a subject at a timing such as after treatment from information related to the state of the subject at a timing such as before the treatment. However, the problems to be solved by the embodiments disclosed in the specification and the drawings are not limited thereto. Problems corresponding to each effect caused by each configuration described in the embodiments described later can also be considered as other problems.

A medical data processing device according to an embodiment includes a first acquisition unit, a calculation unit, and an estimation unit. The first acquisition unit acquires estimated data related to a state of a biological organ at a first timing, and measured data indicating the state of the biological organ at the first timing. The calculation unit calculates a parameter based on the estimated data and the measured data. The estimation unit estimates the state of the biological organ in a predetermined time phase at a second timing different from the first timing based on the parameter and the estimated data in the predetermined time phase.

The following describes embodiments and modifications of the medical data processing device, a medical data processing method, and a medical data processing program in detail with reference to the drawings. The medical data processing device, the medical data processing method, and the medical data processing program according to the present application are not limited to the embodiments and the modifications described below. Each of the embodiments can be combined with another embodiment, the modification, or a conventional technique to the extent that there is no contradiction in processing content. Similarly, each of the modifications can be combined with the embodiment, another modification, or a conventional technique to the extent that there is no contradiction in processing content.

First embodiment

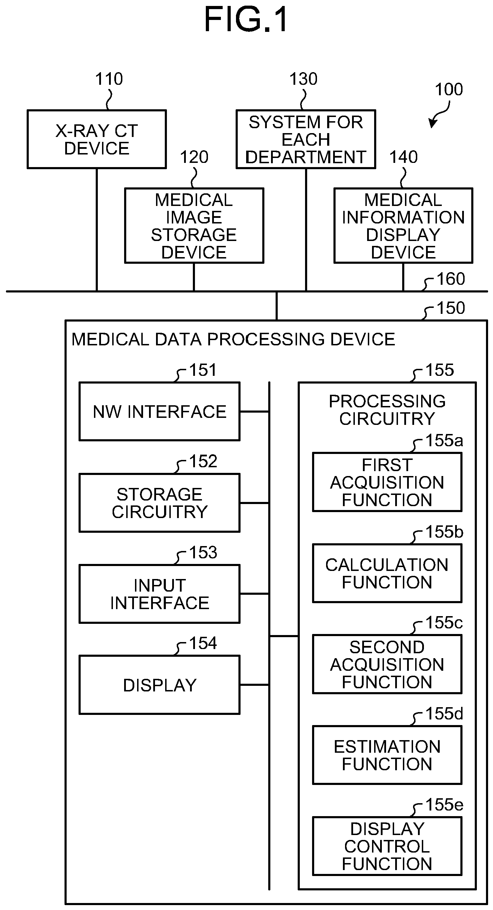

FIG. 1 is a diagram illustrating a configuration example of a medical data processing system and a medical data processing device according to a first embodiment.

As illustrated in FIG. 1, a medical data processing system 100 according to the present embodiment includes an X-ray Computed Tomography (CT) device 110, a medical image storage device 120, a system 130 for each department, a medical information display device 140, and a medical data processing device 150. The devices and systems are connected to each other via a network 160 in a communicable manner.

The X-ray CT device 110 generates a CT image in which a target organ of a subject is delineated. Specifically, the X-ray CT device 110 collects projection data representing distribution of X-rays transmitted through the subject by turning and moving an X-ray tube and an X-ray detector on a circular orbit surrounding the subject. Examples of such a target organ include biological organs such as a mitral valve, a heart, and lungs. The target organ is not limited thereto, but may be any biological organ. The X-ray CT device 110 then generates a CT image based on the collected projection data. The X-ray CT device 110 is an example of a medical image diagnostic device.

The medical data processing system 100 may further include another medical image diagnostic device such as an ultrasonic diagnostic device, a Magnetic Resonance Imaging (MRI) device, an X-ray diagnostic device, a Positron Emission Tomography (PET) device, and a Single Photon Emission Computed Tomography (SPECT) device in addition to the X-ray CT device 110. The ultrasonic diagnostic device generates an ultrasonic image. The MRI device generates an MRI image. The X-ray diagnostic device generates an X-ray image and an angiographic image. The PET device generates a PET image. The SPECT device generates a SPECT image.

Each of the CT image, the ultrasonic image, the MRI image, the X-ray image, the angiographic image, the PET image, and the SPECT image is an example of a medical image.

The medical image storage device 120 keeps various medical images. Specifically, the medical image storage device 120 acquires a CT image from the X-ray CT device 110 via the network 160, and causes the acquired CT image to be stored and kept in storage circuitry in the medical image storage device 120. For example, the medical image storage device 120 is implemented by a Picture Archiving and Communication System (PACS) and the like, and keeps the CT image in a format conforming to Digital Imaging and Communications in Medicine (DICOM). The medical image storage device 120 may similarly acquire another medical image other than the CT image, and cause the acquired other medical image to be stored and kept in the storage circuitry. The medical image storage device 120 is implemented by computer equipment such as a server and a workstation.

The system 130 for each department includes various systems such as an electronic medical chart system, a Hospital Information System (HIS), a Radiology Information System (RIS), a diagnosis report system, a Laboratory Information System (LIS), a rehabilitation department system, a dialysis department system, and a surgery department system. These systems are connected to the respective devices. Various kinds of information are transmitted/received between the respective systems and devices. For example, the medical data processing device 150 receives various kinds of information such as patient information, inspection information, or treatment information transmitted by the systems.

The medical information display device 140 displays various kinds of medical information. For example, the medical information display device 140 acquires medical information such as a CT image and a processing result of image processing from the medical image storage device 120 via the network 160. The medical information display device 140 then causes the acquired medical information to be displayed on a display in the medical information display device 140. The medical information display device 140 also similarly acquires medical information such as another medical image to be displayed on the display. For example, the medical information display device 140 is implemented by computer equipment such as a workstation, a personal computer, or a tablet terminal.

The medical data processing device 150 performs various kinds of image processing on the medical image. For example, the medical data processing device 150 acquires a CT image from the X-ray CT device 110 or the medical image storage device 120 via the network 160. The medical data processing device 150 then performs various kinds of image processing on the acquired CT image. The medical data processing device 150 may similarly acquire another medical image other than the CT image, and may perform various kinds of image processing on the acquired other medical image. The medical data processing device 150 also acquires various kinds of information transmitted by the system 130 for each department via the network 160, and performs various kinds of processing using the acquired various kinds of information. For example, the medical data processing device 150 is implemented by computer equipment such as a server and a workstation.

As illustrated in FIG. 1, the medical data processing device 150 includes a Network (NW) interface 151, storage circuitry 152, an input interface 153, a display 154, and processing circuitry 155.

The NW interface 151 controls transmission and communication of various kinds of information and various kinds of data transmitted and received between the medical data processing device 150 and another device or the system 130 for each department connected to the medical data processing device 150 via the network 160. The NW interface 151 is connected to the processing circuitry 155. The NW interface 151 receives data transmitted from another device or the system 130 for each department. In this case, the NW interface 151 transmits the received data to the processing circuitry 155. The NW interface 151 receives the data transmitted by the processing circuitry 155. In this case, the NW interface 151 transmits the received data to another device or the system 130 for each department. For example, the NW interface 151 is implemented by a network card, a network adapter, a Network Interface Controller (NIC), or the like.

The storage circuitry 152 stores various kinds of data and various computer programs. The storage circuitry 152 is connected to the processing circuitry 155. The storage circuitry 152 stores the data transmitted by the processing circuitry 155 under control of the processing circuitry 155. The data stored in the storage circuitry 152 is read out by the processing circuitry 155. For example, the storage circuitry 152 is implemented by a semiconductor memory element such as a Random Access Memory (RAM) and a flash memory, a hard disk, an optical disc, or the like. The storage circuitry 152 is, for example, a non-transitory computer-readable recording medium recording a computer program.

The input interface 153 receives an input operation for various instructions and various kinds of information from a user such as a doctor. The input interface 153 is connected to the processing circuitry 155. The input interface 153 converts the operation received from the user into an electric signal to be transmitted to the processing circuitry 155. For example, the input interface 153 is implemented by a trackball, a switch button, a mouse, a keyboard, a touch pad that receives an operation when an operation surface thereof is touched, a touch screen in which a display screen and a touch pad are integrated with each other, a non-contact input interface with an optical sensor, a voice input interface, and the like. In the present specification, the input interface 153 does not necessarily include a physical operation component such as a mouse and a keyboard. For example, examples of the input interface 153 include processing circuitry for electric signals that receives an electric signal corresponding to the input operation from an external input appliance that is provided separately from the device, and transmits the electric signal to the processing circuitry 155.

The display 154 displays various kinds of information and various kinds of data. The display 154 is connected to the processing circuitry 155. The display 154 displays various kinds of information and various kinds of data transmitted by the processing circuitry 155 under the control of the processing circuitry 155. For example, the display 154 is implemented by a liquid crystal display, a Cathode Ray Tube (CRT) display, a touch panel, and the like. The display 154 is an example of a display unit.

The processing circuitry 155 controls the entire medical data processing device 150. For example, the processing circuitry 155 performs various kinds of processing in accordance with an operation received from the user via the input interface 153. For example, the processing circuitry 155 also acquires a CT image transmitted by the X-ray CT device 110 or the medical image storage device 120 via the network 160. The processing circuitry 155 then causes the acquired CT image to be stored in the storage circuitry 152. The processing circuitry 155 may similarly acquire another medical image, and may cause the acquired other medical image to be stored in the storage circuitry 152.

Configuration examples of the medical data processing system 100 and the medical data processing device 150 according to the present embodiment have been described above. For example, the medical data processing system 100 and the medical data processing device 150 according to the present embodiment are installed in a medical facility such as a hospital or a clinic, and support a user such as a doctor to develop a treatment plan and the like for various diseases.

For example, the medical data processing device 150 performs (runs) a posttreatment simulation for estimating a state of the biological organ (a shape, movement, a blood flow, a pressure, and the like) after treatment of the subject. In the present embodiment, in performing the posttreatment simulation, the medical data processing device 150 sets various parameters to be used for performing the posttreatment simulation from information about the state of the subject before the treatment while considering differences between individuals for the subject. That is, the medical data processing device 150 sets a more appropriate parameter corresponding to each subject. Thus, the medical data processing device 150 according to the present embodiment can more appropriately estimate the state of the subject after the treatment from the information about the state of the subject before the treatment. Due to this, a patient (subject) is enabled to select appropriate treatment, and a doctor is enabled to shorten a treatment time, so that a prognosis of the subject can be improved.

The following describes a case in which the medical data processing device 150 performs processing by using a CT image in which a mitral valve of the subject (patient) suffering from a valvular disease of the mitral valve is delineated as a target region. Specifically, the following describes a case in which the medical data processing device 150 estimates hemodynamics information and form information of the mitral valve after the treatment from the form information of the mitral valve before the treatment that is obtained from the CT image. Alternatively, the medical data processing device 150 may also perform similar processing on another biological organ other than the mitral valve.

As illustrated in FIG. 1, the processing circuitry 155 of the medical data processing device 150 includes a first acquisition function 155a, a calculation function 155b, a second acquisition function 155c, an estimation function 155d, and a display control function 155e. The first acquisition function 155a is an example of a first acquisition unit. The calculation function 155b is an example of a calculation unit. The second acquisition function 155c is an example of a second acquisition unit. The estimation function 155d is an example of an estimation unit. The display control function 155e is an example of a display control unit.

The processing circuitry 155 is, for example, implemented by a processor. In this case, each of the processing functions described above is stored in the storage circuitry 152 as a computer-executable program (medical data processing program). The processing circuitry 155 then reads out each computer program stored in the storage circuitry 152, and executes the read-out computer program to implement the processing function corresponding to the computer program. In other words, the processing circuitry 155 that has read out each computer program is assumed to have each processing function illustrated in FIG. 1.

The processing circuitry 155 may be configured by combining a plurality of independent processors, and each of the processors may execute each computer program to implement each processing function. The processing functions included in the processing circuitry 155 may be implemented by being distributed or integrated in a single piece of or a plurality of pieces of processing circuitry. The processing functions included in the processing circuitry 155 may be implemented by both of hardware such as circuitry and software. Herein, exemplified is a case in which the computer programs corresponding to the respective processing functions are stored in the single storage circuitry 152, but the computer programs may be stored in a plurality of pieces of storage circuitry in a distributed manner. For example, the computer programs corresponding to the respective processing functions may be stored in a plurality of pieces of the storage circuitry in a distributed manner, and the processing circuitry 155 may be configured to read out, from each piece of the storage circuitry, and execute each computer program.

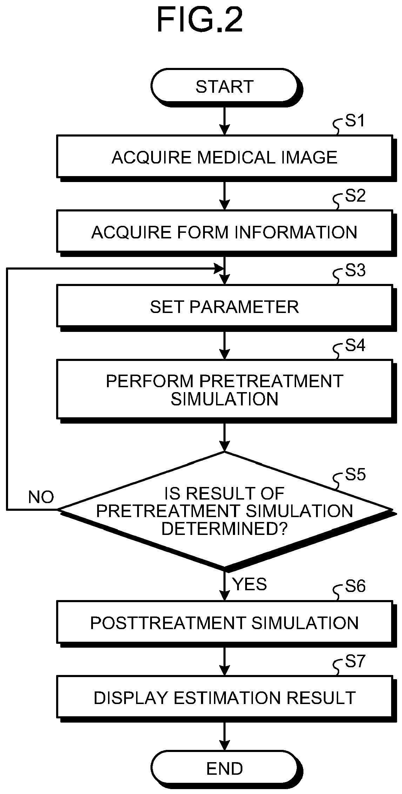

Next, the following describes a processing procedure performed by the medical data processing device 150 with reference to FIG. 2. FIG. 2 is a flowchart illustrating a processing procedure of processing performed by the processing functions included in the processing circuitry 155 of the medical data processing device according to the first embodiment.

The processing circuitry 155 performs the processing illustrated in FIG. 2 in a case of receiving an instruction input by the user via the input interface 153 for performing the processing illustrated in FIG. 2. The processing circuitry 155 may monitor the medical image storage device 120, and may automatically perform the processing illustrated in FIG. 2 in a case in which a new CT image is kept in the medical image storage device 120. Furthermore, the processing circuitry 155 may determine whether the new CT image kept in the medical image storage device 120 satisfies a condition determined in advance, and may perform the processing illustrated in FIG. 2 in a case in which the condition is satisfied.

Step S1

As illustrated in FIG. 2, first, the first acquisition function 155a acquires a CT image of the subject from the X-ray CT device 110 or the medical image storage device 120 at Step S1. In the present embodiment, a type of the medical image as a processing target of the medical data processing device 150 is not limited to the CT image. The medical image as a processing target of the medical data processing device 150 may be any type of medical image including form information of an anatomical structure of the target organ as the processing target. For example, the medical image as the processing target may be another three-dimensional image or two-dimensional image such as an ultrasonic image, an MRI image, an X-ray image, an angiographic image, a PET image, or a SPECT image. The medical image as the processing target may also be a four-dimensional image that is obtained by taking a plurality of images in a time direction. Alternatively, the medical image as the processing target may be a distribution map and the like in a time and/or space direction of one-dimensional measured values of electricity, magnetism, a near infrared ray, and the like obtained by using an electrocardiograph, an electroencephalograph, a magnetoencephalograph, a magnetocardiograph, a near infrared spectroscopy (NIRS) electroencephalograph, or the like.

The following describes an example in which the processing circuitry 155 acquires, at Step S1, a four-dimensional CT image including three-dimensional data corresponding to six phases (six time phases) obtained by imaging a mitral valve at six time points assuming that the mitral valve of the subject delineated in the CT image is the target organ, but the present embodiment is not limited thereto.

Step S2

Next, at Step S2, the first acquisition function 155a specifies a region indicating the target organ in the CT image acquired at Step S1. Hereinafter, the region indicating the target organ is referred to as a target region. That is, in this example, the first acquisition function 155a acquires, as the target region, coordinate information of each pixel in the CT image indicated by the mitral valve on the CT image. In this way, at Step S2, the first acquisition function 155a acquires the form information of the mitral valve. The form information of the mitral valve is an example of actually measured data indicating a state of a form of the mitral valve at a timing before the treatment. The state of the form of the mitral valve is an example of the state of the mitral valve.

For example, the first acquisition function 155a may specify the target region based on designation of the target region that is input by the user via the input interface 153. Specifically, the first acquisition function 155a may specify, as the target region used at Step S3 and succeeding processing, the region designated as the target region by the user. The first acquisition function 155a may also specify the target region based on an anatomical structure delineated in the CT image by a known region extraction technique. Examples of the known region extraction technique include, for example, Otsu's binarization method based on a CT value, a region expansion method, a snake method, a graph cut method, a mean shift method, and the like.

A method of specifying the target region may be any method capable of specifying the target region from the medical image, and is not limited to the method described above. For example, the first acquisition function 155a may estimate and specify the target region based on a shape model of the target region constructed by learning data for learning that is prepared in advance by using a machine learning technique (for example, a machine learning technique including deep learning). Specifically, the first acquisition function 155a estimates the target region by applying the shape model to the CT image and outputting the target region. The target region may be a two-dimensional region or a three-dimensional region.

If the first acquisition function 155a performs processing such as a graph cut method on the entire medical image, calculation cost may become excessively high. Thus, the first acquisition function 155a specifies a region that is related to the target organ, and is larger than the target organ but smaller than the entire medical image. Hereinafter, the region that is related to the target organ, and is larger than the target organ but smaller than the entire medical image is referred to as a relevant region. For example, in a case in which the target organ is a mitral valve, the relevant region is a heart region, a region of a sum of a left atrium and a left ventricle, or the like. The first acquisition function 155a may apply the processing described above only to the specified relevant region to specify the target region. Due to this, the calculation cost can be prevented from being increased.

The first acquisition function 155a may specify the relevant region based on designation of the relevant region that is input by the user via the input interface 153. Specifically, the first acquisition function 155a may specify the region designated as the relevant region by the user to be the relevant region to which the processing described above is applied. The first acquisition function 155a performs the processing of specifying the target region on respective CT images corresponding to a plurality of phases (in this example, data corresponding to six phases) that are imaged at a plurality of time points and acquired at Step S1. That is, the first acquisition function 155a specifies the target region for each time point.

The first acquisition function 155a may individually specify a plurality of characteristic regions or a plurality of regions having different characteristics in the target region. For example, the mitral valve is constituted of two valve lobes including an anterior cusp and a posterior cusp. Thus, the first acquisition function 155a may individually specify the anterior cusp and the posterior cusp.

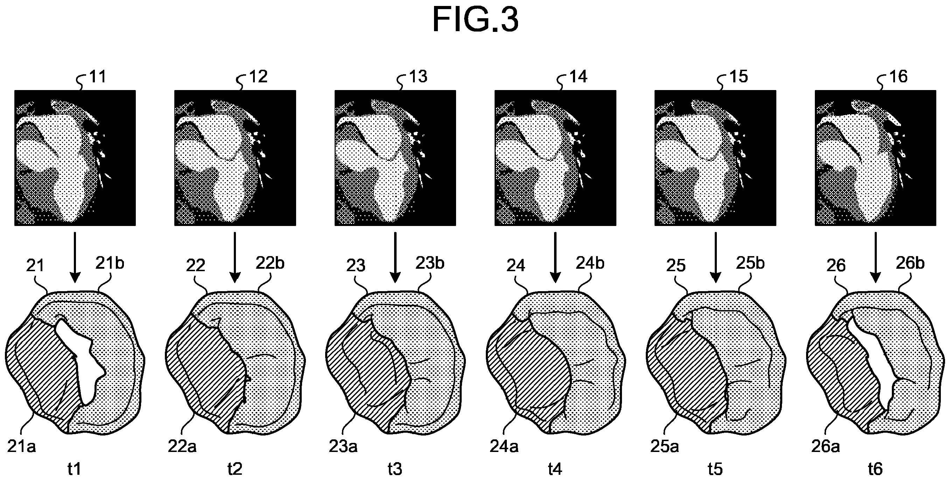

FIG. 3 is a diagram for explaining an example of processing at Step S2 according to the first embodiment. As illustrated in FIG. 3, the first acquisition function 155a specifies respective six regions 21 to 26 of the mitral valve from respective six CT images 11 to 16 that are obtained by performing imaging at six time points from a time point t1 to a time point t6. In the regions 21 to 26 of the mitral valve, regions 21a to 26a represented by hatching with oblique lines are regions indicating the anterior cusp, and regions 21b to 26b represented by hatching with dots are regions indicating the posterior cusp.

Step S3

Next, at Step S3, the calculation function 155b calculates and sets a parameter to be used for a simulation before treatment (pretreatment simulation) performed at Step S4 (described later). The parameter set at Step S3 is a parameter that is not influenced or hardly influenced by the treatment. That is, the parameter set at Step S3 is a parameter that does not vary or varies little before and after the treatment. At Step S3, one or a plurality of parameters are set. That is, the calculation function 155b sets at least one parameter. The parameter includes a boundary condition.

Specifically, the calculation function 155b may set hardness, a thickness, a fiber direction, and the like of a valve as the parameter related to the valve lobe. The calculation function 155b may also set hardness, a length, a width, a connecting position, the number, and the like of a chorda tendinea as a parameter related to the chorda tendinea. The calculation function 155b may also set hardness, a volume, a degree of smoothness of a surface, and the like of each of a blood vessel, a left atrium, a left ventricle, and the like as a parameter related to a heart chamber or a blood vessel. The calculation function 155b may also set viscosity and a flow speed of blood, a cuff pressure (maximal blood pressure, minimal blood pressure), a total amount of blood in the whole body, and the like as a parameter related to the blood. In the present specification, the parameter related to the valve lobe such as the hardness, the thickness, the fiber direction, and the like of the valve described above, and the parameter related to the chorda tendinea such as the hardness, the length, the width, the connecting position, the number, and the like of the chorda tendinea are described as parameters related to a shape or a change in the shape of the mitral valve. The parameter related to the heart chamber or the blood vessel such as the hardness, the volume, the degree of smoothness of a surface, and the like of each of the blood vessel, the left atrium, the left ventricle, and the like described above, and the parameter related to the blood such as the viscosity and the flow speed of blood, the cuff pressure (maximal blood pressure, minimal blood pressure), the total amount of blood in the whole body, and the like are described as parameters related to movement of the mitral valve or a change in movement of the mitral valve. Hereinafter, the parameter related to the shape or a change in the shape of the mitral valve is simply referred to as a "parameter related to the shape", and the parameter related to movement or a change in movement of the mitral valve is simply referred to as a "parameter related to movement". The parameter related to the shape and the parameter related to movement may be classified in any manner, and the present embodiment is not limited thereto. A parameter related to a blood flow may be separately set.

In the present embodiment, for example, biological organs for which the parameter related to the shape is calculated are the mitral valve and the chorda tendinea. In the present embodiment, for example, biological organs for which the parameter related to movement is calculated are the heart chamber, the blood vessel, and the blood. However, the chorda tendinea may also be a biological organ for which the parameter related to movement is calculated. The biological organ for which the parameter related to the shape is calculated is an example of a first biological organ. The biological organ for which the parameter related to movement is calculated is an example of a second biological organ.

The calculation function 155b may calculate and set a parameter from an indirect index based on an algorithm set in advance without directly setting the parameter. For example, the calculation function 155b may calculate and set a volume (capacity) of the left atrium and a volume of the left ventricle as indirect indexes instead of directly setting a parameter indicating a flow speed of the blood passing through the mitral valve. For example, the calculation function 155b calculates a range of pressure applied to an upper side (a left atrium side) and a lower side (left ventricle side) of the mitral valve based on a change amount in a time direction of the volume of the left atrium and the volume of the left ventricle. The calculation function 155b then calculates the flow speed of the blood from the range of pressure, and sets the calculated flow speed of the blood as the parameter. In this way, the calculation function 155b may set the volume of the left atrium and the volume of the left ventricle as indirect indexes for calculating the parameter indicating the flow speed of the blood.

The parameters described above are merely examples, and the type or number of parameters set at Step S3 are not limited to the type or number of the parameters described above. For example, the parameter set by the calculation function 155b may be any parameter that can be used for a simulation of a living body. For example, the parameter related to the shape of the biological organ used in the present embodiment may be a parameter indicating at least one of hardness, a thickness, a fiber direction, a length, a width, a connecting position, and the number of the biological organ. The parameter related to movement of the biological organ used in the present embodiment may be a parameter indicating at least one of hardness, a volume, and a degree of smoothness of a surface of the biological organ, and viscosity, a flow speed, and a total quantity of a fluid (for example, the blood) flowing in the biological organ.

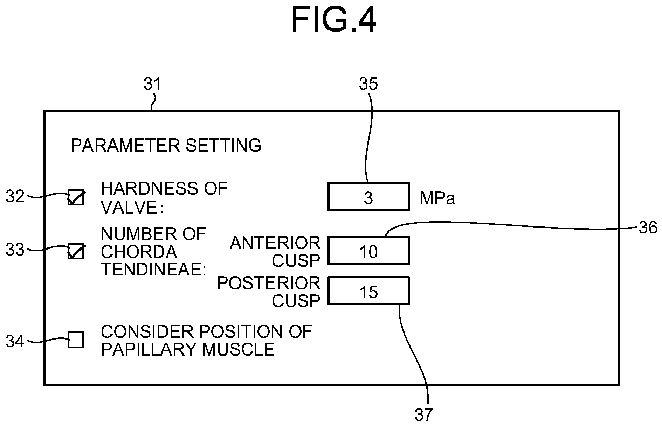

FIG. 4 is a diagram illustrating an example of a user interface according to the first embodiment. FIG. 4 illustrates a screen 31 for parameter setting for setting a parameter as an example of a user interface for setting a parameter. In the present embodiment, the user may be able to manually set a parameter by using the user interface as illustrated in FIG. 4. For example, the calculation function 155b controls the display 154 to display the screen 31 for parameter setting illustrated in FIG. 4. The screen 31 for parameter setting includes three checkboxes 32 to 34 and three textboxes 35 to 37.

The checkbox 32 is used for selecting whether to use a parameter indicating the hardness of the mitral valve for a pretreatment simulation. When the user selects the checkbox 32 via the input interface 153, as illustrated in FIG. 4, the calculation function 155b causes a check mark to be displayed on the checkbox 32, and performs setting so that the parameter indicating the hardness of the mitral valve is used for the pretreatment simulation. On the other hand, in a case in which the checkbox 32 is not selected, the calculation function 155b does not cause the check mark to be displayed on the checkbox 32, and performs setting so that the parameter indicating the hardness of the mitral valve is not used for the pretreatment simulation.

The checkbox 33 is used for selecting whether to use a parameter indicating the number of chorda tendineae for the pretreatment simulation. When the user selects the checkbox 33 via the input interface 153, as illustrated in FIG. 4, the calculation function 155b causes the check mark to be displayed on the checkbox 33, and performs setting so that the parameter indicating the number of chorda tendineae is used for the pretreatment simulation. On the other hand, in a case in which the checkbox 33 is not selected, the calculation function 155b does not cause the check mark to be displayed on the checkbox 33, and performs setting so that the parameter indicating the number of chorda tendineae is not used for the pretreatment simulation.

The checkbox 34 is used for selecting whether to consider a position of a papillary muscle in the pretreatment simulation. When the user selects the checkbox 34 via the input interface 153, the calculation function 155b causes the check mark to be displayed on the checkbox 34, and performs setting so that the position of the papillary muscle is considered in the pretreatment simulation. On the other hand, in a case in which the checkbox 34 is not selected, as illustrated in FIG. 4, the calculation function 155b does not cause the check mark to be displayed on the checkbox 34, and performs setting so that the position of the papillary muscle is not considered in the pretreatment simulation.

The textbox 35 is caused to be in a state capable of receiving an input of a numerical value indicating the hardness of the valve by the user via the input interface 153 when the checkbox 32 is selected. FIG. 4 exemplifies a case in which the user inputs and designates "3" (MPa).

The textbox 36 is caused to be in a state capable of receiving an input of the number of chorda tendineae connected to the anterior cusp by the user via the input interface 153 when the checkbox 33 is selected. FIG. 4 exemplifies a case in which the number "10" of chorda tendineae connected to the anterior cusp is input and designated by the user.

The textbox 37 is caused to be in a state capable of receiving an input of the number of chorda tendineae connected to the posterior cusp by the user via the input interface 153 when the checkbox 33 is selected. FIG. 4 exemplifies a case in which the number "15" of chorda tendineae connected to the posterior cusp is input and designated by the user.

In a case in which various parameters are designated by the user as exemplified in FIG. 4, the calculation function 155b sets three parameters including the hardness "3" (MPa) of the valve designated by the user, the number "10" of the chorda tendineae connected to the anterior cusp, and the number "15" of the chorda tendineae connected to the posterior cusp.

The user interface as described above is merely an example, and any user interface with which the user can set various parameters may be used. For example, the user interface may be configured to enable the user to input various logical expressions (such as AND, OR, and NOT) so that complicated conditions can be set.

The calculation function 155b may individually calculate and set various parameters for the respective characteristic regions in the target region specified at Step S2. The calculation function 155b may automatically calculate and set various parameters by analyzing the CT image acquired at Step S1 instead of manual setting performed by the user. For example, the calculation function 155b may extract a heart chamber region from the CT image using a known region extraction technique, automatically calculate a volume of the heart chamber from the extracted heart chamber region, and set a parameter indicating the calculated volume of the heart chamber. The calculation function 155b may also acquire clinical information from an electronic medical chart system, an HIS, an RIS, and the like, and set a parameter related to the acquired clinical information. Alternatively, the calculation function 155b may set a parameter manually set by the user and a parameter that is automatically set in a mixed manner.

Furthermore, the calculation function 155b may set a constant determined in advance as an initial value of each of the various parameters. For example, the calculation function 155b may set, as the initial value, a constant that is determined based on an attribute of the subject. For example, the calculation function 155b may set different initial values depending on an age or distinction of sex.

The calculation function 155b does not necessarily use items of all parameters defined in advance. For example, the calculation function 155b may enable the user to manually select an item of a parameter to be used on the user interface.

As another example, for a parameter that is automatically acquired by performing image processing and the like on the CT image, the calculation function 155b may calculate reliability of the image processing, and determine whether the reliability is low. Specifically, the calculation function 155b may determine whether the reliability is low by determining whether the reliability is equal to or smaller than a threshold. In a case in which the reliability is equal to or smaller than the threshold, the calculation function 155b may perform any of three pieces of processing described below. For example, as the first processing, the calculation function 155b may set, as the parameter, a constant determined in advance instead of a calculated value obtained by image processing. As the second processing, the calculation function 155b may cause the display 154 to display a screen for prompting the user to manually set the parameter. As the third processing, the calculation function 155b may perform setting so that the item of the parameter that is automatically acquired is not used. In a case in which the reliability is larger than the threshold, the calculation function 155b sets the automatically acquired parameter.

The following describes a case in which, although the calculation function 155b tries to acquire the parameter from various systems such as the electronic medical chart system, the HIS, and the RIS as described above, the parameter cannot be acquired from the various systems. In this case, the calculation function 155b may perform processing similar to the processing that is performed in a case in which the reliability is equal to or smaller than the threshold as described above. For example, the calculation function 155b may set a constant determined in advance as the parameter. The calculation function 155b may also cause the display 154 to display a screen for prompting the user to manually set the parameter. The calculation function 155b may also perform setting so that the item of the parameter that cannot be acquired from various systems is not used.

The following describes a case in which the calculation function 155b sets, as the parameters, the hardness of the valve, the number of chorda tendineae (the number of chorda tendineae respectively connected to the anterior cusp and the posterior cusp), the volume (capacity) of the left atrium and the volume (capacity) of the left ventricle in each of a diastole phase and a systole phase, and the cuff pressure (maximal blood pressure, minimal blood pressure). The calculation function 155b sets "3" MPa as an initial value of the hardness of the valve. The calculation function 155b also sets "10" as an initial value of the number of chorda tendineae connected to the anterior cusp, and sets "15" as an initial value of the number of chorda tendineae connected to the posterior cusp. The calculation function 155b also performs region extraction processing on the CT image acquired at Step S1 to acquire the volume of the left atrium and the volume of the left ventricle in each of the diastole phase and the systole phase, and sets the acquired four volumes in total. The calculation function 155b also acquires the cuff pressure from the electronic medical chart system, and sets the acquired cuff pressure. As described above, in the present example, the respective parameters of the hardness of the valve and the number of chorda tendineae are parameters related to the shape, and the respective parameters of the volume of the left atrium and the volume of the left ventricle in each of the diastole phase and the systole phase, and the cuff pressure are parameters related to movement.

Step S4

Next, at Step S4, the first acquisition function 155a performs the pretreatment simulation based on the parameters set at Step S3, and acquires estimated data of the target organ before the treatment of the subject. That is, the first acquisition function 155a acquires the estimated data by performing the simulation for acquiring the estimated data. In the present embodiment, the first acquisition function 155a simulates movement of the mitral valve before the treatment. The first acquisition function 155a performs the pretreatment simulation using a known method. For example, the first acquisition function 155a performs the pretreatment simulation using methods such as the finite element method, the finite difference method, the immersed boundary method, etc.

The following exemplifies a case in which the first acquisition function 155a performs the pretreatment simulation by using the finite element method, but the method of performing the pretreatment simulation is not limited thereto. The first acquisition function 155a may use any method capable of estimating information related to an operation of an object or a fluid. For example, the first acquisition function 155a may estimate the shape of the target region before the treatment based on a shape model constructed by learning data for learning that is prepared in advance by using a machine learning technique (for example, a machine learning technique including deep learning). Specifically, the first acquisition function 155a applies the shape model to the CT image and outputs the shape of the target region before the treatment to estimate the shape of the target region before the treatment.

The following describes an example of specific processing at Step S4. First, the first acquisition function 155a acquires a target region at one time point from target regions at a plurality of time points (six time points in the present embodiment) specified at Step S2. For example, the first acquisition function 155a acquires the region 21 of the mitral valve at the time point t1 illustrated in FIG. 3. The first acquisition function 155a then sets a plurality of calculation grids (meshes) based on conditions such as the number, a size, a shape, and an element determined in advance for the acquired region 21 of the mitral valve. The first acquisition function 155a then applies a mathematical model or a physical model that is set based on the parameter set at Step S3 to each of the calculation grids, and estimates shapes (forms) of the mitral valve at five time points from the time point t2 to the time point t6.

The first acquisition function 155a may estimate only the shapes at the five time points from the time point t2 to the time point t6, or may estimate shapes at finer time intervals (for example, a time point between the time point t1 and the time point t2 and the like). The first acquisition function 155a may also estimate shapes at the respective time points from the time point t2 to the time point t6 based on only the shape at the time point t1, the parameter set at Step S3, and information about a time interval between the time point t1 and a time point as an estimation target. In estimating the shape at each time point of a plurality of time points continuous to each other in a time direction, the first acquisition function 155a may estimate a shape at a time point next to a certain time point based on a shape estimated at the certain time point. That is, the first acquisition function 155a may estimate a shape at a time point t(N+1) based on a shape at a time point tN (N is an integral number from 1 to 5) and the parameter set at Step S3.

In performing the simulation, the first acquisition function 155a may use not only the shape of the mitral valve and the parameter set at Step S3 but also clinical information of the subject as a target. For example, the first acquisition function 155a may perform control to specify, from a system such as an electronic medical chart system, an HIS, and an RIS, and use the clinical information as the target. The first acquisition function 155a may also use information obtained through inquiry, hearing, or the like with the subject input by the user via the input interface 153.

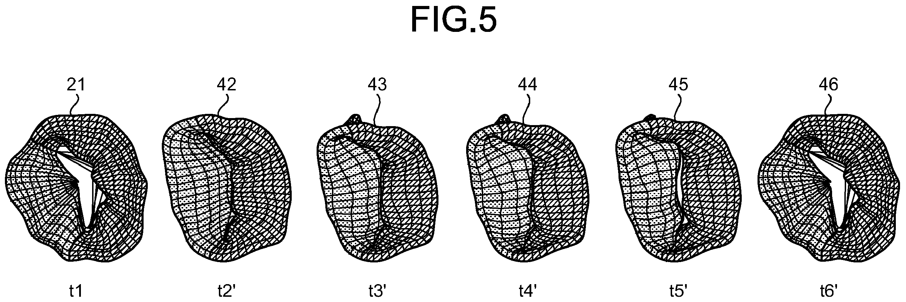

FIG. 5 is a diagram for explaining an example of processing at Step S4 according to the first embodiment. FIG. 5 illustrates examples of shapes of the mitral valve at respective time points from a time point t2' to a time point t6' that are estimated by the first acquisition function 155a at Step S4 based on the shape of the region 21 of the mitral valve at the time point t1 obtained at Step S2. The respective time points from the time point t2' to the time point t6' are time points for the same cardiac phase as a cardiac phase of the respective time points from the time point t2 to the time point t6. As illustrated in FIG. 5, at Step S4, the first acquisition function 155a estimates a shape 42 of the mitral valve at the time point t2'. Similarly, at Step S4, the first acquisition function 155a estimates shapes 43, 44, 45, and 46 of the mitral valve at the respective time points from the time point t3' to the time point t6'.

Step S5

Next, at Step S5, the calculation function 155b performs evaluation using an evaluation index set in advance based on the shape estimated at Step S4 (hereinafter, referred to as an estimated shape) and the shape of the target region specified at Step S2 (hereinafter, referred to as an actually measured shape). Herein, the estimated shape is an example of estimated data related to the state of the mitral valve at a timing before the treatment. The actually measured shape is an example of actually measured data indicating the state of the mitral valve at a timing before the treatment. The timing before the treatment is an example of the first timing.

For example, the calculation function 155b calculates an evaluation value using the evaluation index based on an error in an overall shape between the estimated shape and the actually measured shape. The calculation function 155b calculates the error in the overall shape as the evaluation value in accordance with the following expression (1).

$$\frac{1}{p}\sum\nolimits_{n=1}^{p}\left\{\frac{1}{|S(S_n)| + |S(G_n)|}\left(\sum_{s_{S_n}\in S(S_n)} d_m\left(s_{S_n}, S(G_n)\right) + \sum_{s_{G_n}\in S(G_n)} d_m\left(s_{G_n}, S(S_n)\right)\right)\right\}$$

$$(1)$$

Herein, p represents the number of phases, and $d_m(a, b)$ represents a function for calculating a minimum Euclidean distance between a point a and a plane b. $S(S_n)$ represents the estimated shape in the n-th phase, and $S(G_n)$ represents the actually measured shape in the n-th phase. $s_{S_n}$ represents a point constituting the estimated shape in the n-th phase. $s_{G_n}$ represents a point constituting the actually measured shape in the n-th phase. $|S(S_n)|$ represents the number of points constituting the estimated shape in the n-th phase. $|S(G_n)|$ represents the number of points constituting the actually measured shape in the n-th phase.

For example, the calculation function 155b calculates errors (differences) between the shapes 42, 43, 44, 45, and 46 of the mitral valve at the respective time points from the time point t2' to the time point t6' and the shapes of the regions 22, 23, 24, 25, and 26 of the mitral valve at the respective time points from the time point t2 to the time point t6 in accordance with the expression (1). In this case, the number p of phases indicates "5". In this way, the calculation function 155b calculates the evaluation value based on the point and the plane constituting the estimated shape, and the point and the plane constituting the actually measured shape.

The evaluation index described above is merely an example, and the evaluation index used in the present embodiment is not limited to the evaluation index described above. In the example described above, described is a case in which the calculation function 155b calculates the evaluation value based on the overall shape. That is, described is a case in which the calculation function 155b calculates, as the evaluation value, the error between the overall shape of the mitral valve estimated at Step S4 and the overall shape of the mitral valve (target region) specified at Step S2.

However, the calculation function 155b may also calculate the evaluation value based on the shape of the characteristic region. For example, the calculation function 155b may calculate, as the evaluation value, an error between a shape of an opening or a shape of a valve annulus of the mitral valve estimated at Step S4, and a shape of an opening or a shape of a valve annulus of the mitral valve specified at Step S2.

The calculation function 155b may also calculate the evaluation value based on a position of characteristic coordinates. For example, the calculation function 155b may calculate the evaluation value based on a position of a commissure. Specifically, the calculation function 155b may calculate, as the evaluation value, an error between positions of an anterior commissure and a posterior commissure of the mitral valve estimated at Step S4, and positions of an anterior commissure and a posterior commissure of the mitral valve specified at Step S2.

In the example described above, described is a case in which the calculation function 155b calculates the evaluation value based on an average value of a plurality of errors at a plurality of time points, but the evaluation value may be calculated based on an average value of errors at characteristic time points. For example, the calculation function 155b may calculate the evaluation value based on an average value of errors in the systole phase and the diastole phase. The calculation function 155b may also calculate the evaluation value by setting a weight to an error at each time point. The calculation function 155b may also combine a plurality of evaluation indexes to perform evaluation. The calculation function 155b may also select the evaluation index to be used from among the evaluation indexes in accordance with the subject as a target, a type of the medical image, a type of the target organ, and the like.

After calculating the evaluation value using the evaluation index set in advance, the calculation function 155b compares the calculated evaluation value with a reference value to determine whether the evaluation value satisfies the reference value. As the evaluation value calculated by the expression (1) becomes smaller, the error becomes smaller. Thus, in a case of calculating the evaluation value by the expression (1), the calculation function 155b determines whether the evaluation value is equal to or smaller than the reference value. In this case, in a case in which the evaluation value is equal to or smaller than the reference value, the calculation function 155b determines that the evaluation value satisfies the reference value. On the other hand, in a case in which the evaluation value is larger than the reference value, the calculation function 155b determines that the evaluation value does not satisfy the reference value. The reference value may be determined in advance, or may be set by the user using the user interface. Herein, for example, "the evaluation value satisfies the reference value" means that "the evaluation value satisfies a standard determined in advance", and "the evaluation value does not satisfy the reference value" means that "the evaluation value does not satisfy the standard determined in advance".

If the evaluation value satisfies the reference value (Yes at Step S5), the calculation function 155b causes the storage circuitry 152 to store and keep the parameter set at Step S3 at this point. In this way, the calculation function 155b calculates a parameter based on the estimated shape and the actually measured shape, the parameter to be used for the posttreatment simulation performed at Step S6. For example, the calculation function 155b calculates, as a parameter used for the posttreatment simulation performed at Step S6, a parameter used for estimating the estimated shape in a case in which the evaluation value calculated based on the estimated shape and the actually measured shape satisfies the standard determined in advance. The calculated parameter is a parameter for bringing the estimated data closer to the actually measured data and for adjusting the simulation performed at Step S4. Specifically, the calculation function 155b calculates, as the parameter used for the posttreatment simulation performed at Step S6, a parameter used for estimating the estimated shape in a case in which the estimated shape is similar to the actually measured shape. More specifically, the calculation function 155b calculates, as the parameter used for the posttreatment simulation performed at Step S6, a parameter used for estimating the estimated shape in a case in which a difference between the estimated shape and the actually measured shape is equal to or smaller than a threshold. The calculation function 155b then determines the calculated parameter to be the parameter used for performing the posttreatment simulation at Step S6 described later. The calculation function 155b then advances the process to Step S6. On the other hand, if the evaluation value does not satisfy the reference value (No at Step S5), the calculation function 155b returns the process to Step S3.

In a case of returning the process to Step S3, the calculation function 155b sets the parameter again at Step S3. For example, the calculation function 155b newly calculates a parameter by changing at least one of a plurality of parameters at Step S3, and sets the calculated parameter. The calculation function 155b may also set a value designated by the user via the user interface as the parameter again. The calculation function 155b may automatically adjust the parameter based on a value of the parameter that is previously set. For example, the calculation function 155b may set various parameters again by varying, by a certain value or a certain ratio, values of the various parameters that are previously set.

The following describes a case in which the first acquisition function 155a and the calculation function 155b repeatedly perform Steps S3 to S5 multiple times. In this case, the calculation function 155b repeatedly changes the parameter used for estimating the estimated shape until the evaluation value calculated based on the estimated shape and the actually measured shape satisfies the reference value. Specifically, the calculation function 155b repeatedly changes the parameter used for estimating the estimated shape until the estimated shape becomes similar to the actually measured shape. More specifically, the calculation function 155b repeatedly changes the parameter used for estimating the estimated shape until the difference between the estimated shape and the actually measured shape becomes equal to or smaller than the threshold.

In a case in which Steps S3 to S5 are repeatedly performed multiple times, the calculation function 155b may estimate a parameter with which the calculated evaluation value comes closer to the reference value from among the parameters, and preferentially adjust the estimated parameter instead of equally adjusting all of the parameters. For example, the calculation function 155b may vary the parameter one by one at Step S3 that is repeatedly performed, estimate a parameter that is the most effective to cause the evaluation value to be closer to the reference value, and preferentially adjust the estimated parameter. That is, the calculation function 155b preferentially changes a parameter with which the evaluation value calculated based on the estimated shape and the actually measured shape comes closer to the reference value as compared with the other parameters among the parameters used for estimating the estimated shape. Every time the calculation function 155b preferentially changes the parameter with which the evaluation value comes closer to the reference value as compared with the other parameters, the first acquisition function 155a estimates the estimated shape using the changed parameter.

The calculation function 155b may keep various parameters that are once set in the storage circuitry 152, and perform control so that parameters as a combination of the same values are not set again. The calculation function 155b may control a parameter that is automatically acquired through image processing and the like to be a fixed value that cannot be adjusted. For example, the calculation function 155b may control the parameters such as the volume of the left atrium and the volume of the left ventricle in each of the diastole phase and the systole phase, and the cuff pressure acquired from a system such as an electronic medical chart system to be fixed values that cannot be adjusted.

Even in a case in which the evaluation value does not satisfy the reference value, the calculation function 155b may advance the process to Step S6 when the processing from Step S3 to Step S5 is repeated by a predetermined number of times or for a predetermined processing time. At this point, the calculation function 155b may keep, in the storage circuitry 152, a parameter at the time when the evaluation value closest to the reference value in the repeated processing or a parameter that is lastly calculated, and advance the process to Step S6. In such a case, for example, the processing from Step S3 to Step S5 is performed multiple times. At Step S4 in each piece of the processing, the first acquisition function 155a acquires the estimated shape by estimating the estimated shape by using a parameter different from the parameter used at Step S3 in the previous processing. Thus, at Step S4 in a plurality of pieces of the processing, the first acquisition function 155a acquires a plurality of the estimated shapes by estimating the estimated shapes using a plurality of different parameters. At Step S5 in each piece of the processing, the calculation function 155b determines whether the evaluation value calculated based on the estimated shape acquired at Step S3 and the actually measured shape acquired at Step S2 satisfies the reference value. Thus, at Steps S3 to S5 in a plurality of pieces of the processing, the calculation function 155b calculates the parameter used for estimating the state of the mitral valve at Step S6 based on the estimated shapes acquired at Step S4 and the actually measured shapes acquired at Step S2 in a plurality of pieces of the processing.

Alternatively, the calculation function 155b may perform control to cause the display 154 to display a warning screen (not illustrated) and to stop the processing without advancing the process to Step S6. When the warning screen is displayed, the user can grasp the reason why the processing is stopped is that the evaluation value does not satisfy the reference value. When the processing is stopped, it is possible to prevent the posttreatment simulation from being performed at Step S6 (described later) although the evaluation value does not satisfy the reference value.

The medical data processing device 150 according to the first embodiment can set the parameter suitable for the subject as a target through the processing from Step S3 to Step S5.

Step S6

At Step S6, the second acquisition function 155c and the estimation function 155d perform a simulation after the treatment (posttreatment simulation) using the parameter that is set at Step S3 when the evaluation value satisfies the reference value at Step S5. That is, the second acquisition function 155c and the estimation function 155d perform the posttreatment simulation using the parameter determined at Step S5. For example, the second acquisition function 155c and the estimation function 155d perform the posttreatment simulation using a known method similarly to Step S4. The following describes a case in which the second acquisition function 155c and the estimation function 155d perform the posttreatment simulation when treatment is performed on the mitral valve with a clip used for repair for mitral regurgitation.

Specifically, first, the second acquisition function 155c acquires a target region at one time point from the target regions at the time points specified at Step S2. For example, the second acquisition function 155c acquires the region 21 of the mitral valve at the time point t1 illustrated in FIG. 3. The second acquisition function 155c then sets calculation grids to the region 21 of the mitral valve based on conditions such as the number, a size, a shape, an element, and the like determined in advance. The second acquisition function 155c may set the calculation grids to the region 21 of the mitral valve using a setting similar to the setting of the calculation grids at Step S4, or may set the calculation grids to the region 21 of the mitral valve using a setting different from the setting of the calculation grids at Step S4. The second acquisition function 155c may also perform processing described below by using the region 21 of the mitral valve to which the calculation grids are set at Step S4 as it is without setting the calculation grids at Step S6.

The second acquisition function 155c then applies, to each of the calculation grids set to the region 21 of the mitral valve, a mathematical model or a physical model that is set based on a parameter related to the clip set in advance based on a known size, weight, tension, or the like of the clip, and the parameter related to the shape determined at Step S5. The second acquisition function 155c then estimates the shape of the mitral valve after the treatment at a time point $t_a1$ in the same cardiac phase as the cardiac phase at the time point t1 by using the mathematical model or the physical model applied to each of the calculation grids set to the region 21 of the mitral valve. In this way, the second acquisition function 155c can perform the posttreatment simulation with higher accuracy by using the parameter related to the shape at the time of performing the posttreatment simulation. Specifically, for example, the second acquisition function 155c can estimate the shape of the mitral valve after the treatment at the time point $t_a1$ with high accuracy.

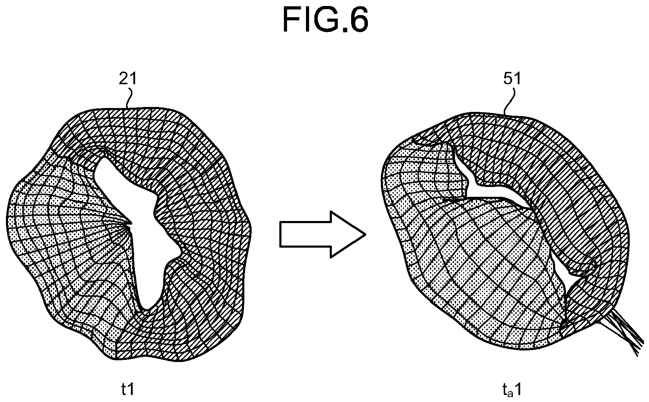

FIG. 6 is a diagram for explaining an example of processing performed by the second acquisition function 155c according to the first embodiment. As illustrated in FIG. 6, at Step S6, the second acquisition function 155c estimates a shape 51 of the mitral valve after the treatment at the time point $t_a1$ from the shape of the region 21 of the mitral valve before the treatment at the time point t1. In this way, the second acquisition function 155c acquires the shape 51 of the mitral valve at the time point $t_a1$ at a timing after the treatment different from the timing before the treatment based on the parameter related to the shape determined at Step S5 and the estimated shape at the time point t1. The shape 51 of the mitral valve is an example of state data related to the state of the mitral valve. The timing after the treatment is an example of a second timing. The time point t1 and the time point $t_a1$ are examples of a predetermined time phase. At Step S6, instead of the second acquisition function 155c, the estimation function 155d may perform the processing described above to estimate the shape 51 of the mitral valve after the treatment at the time point $t_a1$.

The user may optionally set the size of the clip or a position on the mitral valve at which the clip is disposed by using the user interface. For example, the second acquisition function 155c may set, as the parameter, the size of the clip or the position on the mitral valve at which the clip is disposed that is designated by the user via the user interface. The second acquisition function 155c may then estimate the shape 51 of the mitral valve after the treatment at the time point $t_a1$ using the size of the clip or the position on the mitral valve at which the clip is disposed that is set as the parameter.

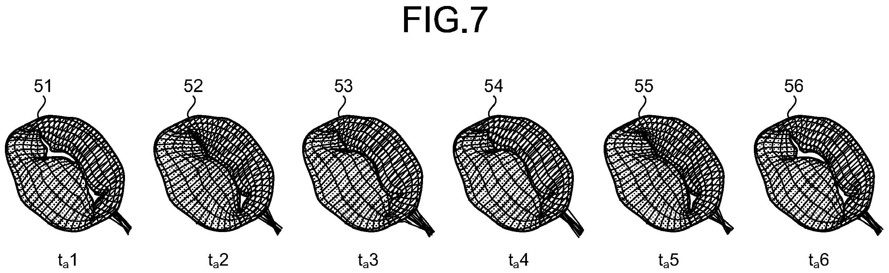

Next, the estimation function 155d applies the parameter related to movement determined at Step S5 to the shape 51 of the mitral valve at the time point $t_a1$, and estimates the shape of the mitral valve at another time point after the treatment. FIG. 7 is a diagram for explaining an example of processing performed by the estimation function 155d according to the first embodiment. As illustrated in FIG. 7, the estimation function 155d estimates shapes 52 to 56 of the mitral valve at other time points after the treatment (five time points from a time point $t_a2$ to a time point $t_a6$). That is, the estimation function 155d estimates the state of the mitral valve at the timing after the treatment over a plurality of time phases based on the shape 51 of the mitral valve at the time point $t_a1$ and the parameter related to movement determined at Step S5. The estimation function 155d may estimate the state of the mitral valve over at least one cycle of time phase with respect to movement of the mitral valve. Herein, a time point $t_aL$ (L is an integral number from 1 to 6) is a time point in the same cardiac phase as that of a time point tL. At Step S6, the estimation function 155d may apply both of the parameter related to movement determined at Step S5 and the parameter related to the shape to the shape 51 of the mitral valve at the time point $t_a1$, and estimate the shape of the mitral valve at another time point after the treatment. Alternatively, the estimation function 155d may apply the parameter related to the shape determined at Step S5 to the shape 51 of the mitral valve at the time point $t_a1$, and estimate the shape of the mitral valve at another time point after the treatment.

In this way, at Step S6, the medical data processing device 150 performs the posttreatment simulation using the parameter suitable for the subject as a target. Thus, the medical data processing device 150 can perform the posttreatment simulation while considering differences between individuals.

Step S7

At Step S7, the display control function 155e controls the display 154 to display an estimation result and the like obtained by the estimation function 155d. For example, the display control function 155e controls the display 154 to display the shapes 51 to 56 of the mitral valve at the six time points from the time point $t_a1$ to the time point $t_a6$. Thus, the medical data processing device 150 according to the present embodiment can cause the user to grasp the state of the subject after the treatment that is more appropriately estimated from information about the state of the subject before the treatment. Due to this, the patient (subject) is enabled to select appropriate treatment, and the doctor is enabled to shorten a treatment time, so that a prognosis of the subject can be improved.

The first embodiment describes a case in which the medical data processing device 150 performs the posttreatment simulation in a case of performing treatment by which the shape of the mitral valve is changed due to the clip held as a treatment device. However, the first embodiment is not limited thereto. For example, the medical data processing device 150 may perform the posttreatment simulation in a case of performing treatment by which the shape of the mitral valve (that is, the target organ) itself is changed, the treatment including valve formation as surgery. In this case, the medical data processing device 150 may estimate the shape of the mitral valve by deforming the shape of part of the mitral valve at one time point by excision or suture, and apply the parameter to the shape of the mitral valve to estimate the shape of the mitral valve after the treatment at another time point.

The medical data processing device 150 may also perform the posttreatment simulation in a case of performing treatment for changing a physical property value (for example, viscosity) of the blood without changing the form of the mitral valve as in pharmacotherapy. In this case, the medical data processing device 150 may estimate the state after the treatment by changing only the physical property value of the blood with respect to the parameter determined at Step S5.

A device for performing the processing at each step may be implemented as each processing unit in the single medical data processing device 150, or may be implemented as an independent device. Each piece of software may perform the processing at each step. In this case, the software that performs the processing may operate on a server via a network including a cloud.

Second embodiment

The above first embodiment describes a case in which the second acquisition function 155c estimates, at Step S6, the shape 51 of the mitral valve after the treatment at the time point $t_a1$ using the parameter related to the shape determined at Step S5. However, the second acquisition function 155c may estimate the shape of the mitral valve after the treatment at the time point $t_a1$ using the parameter related to movement instead of the parameter related to the shape. Thus, such an embodiment is described as a second embodiment. In the description about the second embodiment, differences from the first embodiment described above are mainly described, and the description about the same configuration as that in the first embodiment described above may be omitted.

In the second embodiment, the second acquisition function 155c applies, to each of the calculation grids set to the region 21 of the mitral valve, the mathematical model or the physical model that is set based on the parameter related to the clip set in advance based on a known size, weight, tension, and the like of the clip, and the parameter related to movement determined at Step S5. The second acquisition function 155c then estimates the shape of the mitral valve after the treatment at the time point $t_a1$ by using the mathematical model or the physical model applied to each of the calculation grids set to the region 21 of the mitral valve. In this way, in the second embodiment, the second acquisition function 155c can estimate the shape of the mitral valve after the treatment from the time point $t_a2$ to the time point $t_a6$ with high accuracy by using the parameter related to movement at the time of performing the posttreatment simulation.

Third embodiment

At Step S6, the second acquisition function 155c may use both of the parameter related to the shape and the parameter related to movement determined at Step S5 to estimate the shape 51 of the mitral valve after the treatment at the time point $t_a1$. Thus, such an embodiment is described as a third embodiment. In the description about the third embodiment, differences from the first embodiment described above are mainly described, and the description about the same configuration as that in the first embodiment described above may be omitted.

In the third embodiment, the second acquisition function 155c applies, to each of the calculation grids set to the region 21 of the mitral valve, the mathematical model or the physical model that is set based on the parameter related to the clip set in advance based on a known size, weight, tension, and the like of the clip, and the parameter related to the shape and the parameter related to movement determined at Step S5. The second acquisition function 155c then estimates the shape of the mitral valve after the treatment at the time point $t_a1$ by using the mathematical model or the physical model applied to each of the calculation grids set to the region 21 of the mitral valve. In this way, in the third embodiment, the second acquisition function 155c can estimate the shapes of the mitral valve after the treatment from the time point $t_a1$ to the time point $t_a6$ with high accuracy by using the parameter related to the shape and the parameter related to movement at the time of performing the posttreatment simulation. At Step S6, the second acquisition function 155c may estimate the shape 51 of the mitral valve after the treatment at the time point $t_a1$ without using the parameter determined at Step S5.

First modification

The above embodiments describe a case in which the medical data processing device 150 acquires the form information at Step S2, estimates the form information at Step S4, and adjusts the parameter used for the pretreatment simulation so that the form information acquired at Step S5 becomes similar to the estimated form information. However, the information used by the medical data processing device 150 is not limited to the form information. The medical data processing device 150 may perform similar processing by using fluid information of a blood flow instead of the form information. Thus, such a modification is described as a first modification. In the description about the first modification, differences from the embodiments described above are mainly described, and the description about the same configuration as that in the embodiments described above may be omitted.

In the first modification, at Step S1, the first acquisition function 155a acquires an ultrasonic image, an MRI image, a contrast CT image of a plurality of time phases, or the like. At Step S2, the first acquisition function 155a acquires a state of the blood flow.

Specifically, in a case in which an ultrasonic image is acquired at Step S1, the first acquisition function 155a acquires fluid information of the blood flow from the ultrasonic image based on Doppler method at Step S2. In a case in which an MRI image is acquired at Step S1, the first acquisition function 155a performs known four-dimensional fluid analysis (four-dimensional flow analysis) on the MRI image to acquire the fluid information at Step S2. In a case in which contrast CT images of a plurality of time phases are acquired at Step S1, the first acquisition function 155a analyzes changes of a CT value at various blood vessel positions in the contrast CT images of the time phases to acquire the fluid information at Step S2. At Step S4, the first acquisition function 155a estimates the fluid information. At Step S5, the calculation function 155b adjusts the parameter used for the pretreatment simulation so that the acquired fluid information becomes similar to the estimated fluid information.

That is, in the first modification, the first acquisition function 155a acquires, as the state of the biological organ at the first timing, the estimated data related to the state of the blood flow in the biological organ, and the actually measured data indicating the state of the blood flow in the biological organ. The second acquisition function 155c then estimates the state of the blood flow in the biological organ in a predetermined time phase at the second timing. In the first modification, instead of the second acquisition function 155c, the estimation function 155d may estimate the state of the blood flow in the biological organ in a predetermined time phase at the second timing similarly to the first embodiment. The estimation function 155d then estimates the state of the blood flow in the biological organ over a plurality of time phases after the predetermined time phase at the second timing. Thus, the medical data processing device 150 according to the first modification can more appropriately estimate the state of the subject at a timing such as after the treatment from the information about the state of the subject at a timing such as before the treatment.

The fluid information may be a backward flow amount or a forward flow amount of the blood flow in the mitral valve. The medical data processing device 150 according to the first modification may further acquire the form information at Step S2, and estimate the form information at Step S4 similarly to the embodiments described above. The calculation function 155b may adjust the parameter so that the acquired fluid information becomes similar to the estimated fluid information, and the acquired form information becomes similar to the estimated form information. In this case, a simulation may be performed by fluid structure interaction (FSI analysis) at Step S4 or Step S6.

Second modification

In the above embodiments, described is a case in which the first acquisition function 155a acquires, at Step S2, the form information from the CT images at all the time points acquired at Step S1 (in this example, the CT images corresponding to the six phases). However, at Step S2, the first acquisition function 155a may acquire the form information from only CT images in part of the phases among the CT images corresponding to the phases acquired at Step S1. Such a modification is described as a second modification. In the description about the second modification, differences from the embodiments described above are mainly described, and the description about the same configuration as that in the embodiments described above may be omitted.

For example, the first acquisition function 155a may acquire the form information only from CT images at two time points including a telesystolic phase and a telediastolic phase. In this case, the first acquisition function 155a may determine the time point for acquiring the form information based on a condition determined in advance, or may cause the user to set the time point using the user interface. Alternatively, the first acquisition function 155a may automatically determine and specify the time point for acquiring the form information by performing image processing on the CT image. For example, the first acquisition function 155a may specify the CT image at the time point in the telesystolic phase or the telediastolic phase based on information about electrocardiographic synchronization recorded in a DICOM header. The first acquisition function 155a may also calculate the capacity of the left ventricle by performing image processing on the CT image, and specify the CT image at the time point in the telesystolic phase or the telediastolic phase based on the calculated capacity of the left ventricle.

That is, in the second modification, the first acquisition function 155a acquires the estimated shape and the actually measured shape by using the CT images of at least two time phases among the CT images of three or more time phases before the treatment, the CT images in which the mitral valve is delineated. Thus, the medical data processing device 150 according to the second modification can more appropriately estimate the state of the subject at a timing such as after the treatment from the information about the state of the subject at a timing such as before the treatment with smaller throughput.

Third modification

In the embodiments described above, described is a case in which the items of parameters set at Step S3 are boundary conditions and parameters for the target organ or the target subject. In the embodiments described above, described is a case in which a condition set in advance is used as a calculation parameter for performing the pretreatment simulation and the posttreatment simulation. However, the calculation function 155b may change the calculation parameter for performing the pretreatment simulation and the posttreatment simulation. Thus, such a modification is described as a third modification. In the description about the third modification, differences from the embodiments described above are mainly described, and the description about the same configuration as that in the embodiments described above may be omitted.

For example, the calculation function 155b may set setting conditions for the calculation grids (for example, positions, the number, and shapes of meshes, and types of elements (primary, secondary, and the like)), and convergence conditions (the number of steps of processing (loop frequency), a time, and the like). In this case, a determination condition for the calculation parameter may be set at Step S5, and the calculation function 155b may set the calculation parameter again based on the determination condition at the time of returning the process to Step S3. For example, in a case in which a calculation time is longer than a certain time, the calculation function 155b may change the setting condition for the calculation grid to shorten the calculation time at the time of returning the process to Step S3. For example, the calculation function 155b may coarsen the calculation grids, or reduce the number of elements.

That is, in the third modification, the calculation function 155b further changes the calculation parameter for performing the pretreatment simulation. The first acquisition function 155a then further performs the pretreatment simulation based on the changed calculation parameter. Thus, the medical data processing device 150 according to the third modification sets a more appropriate calculation parameter corresponding to each subject. Accordingly, the medical data processing device 150 according to the third modification can more appropriately estimate the state of the subject after the treatment from the information about the state of the subject before the treatment. Due to this, the patient (subject) is further enabled to select appropriate treatment, and the doctor is enabled to shorten a treatment time, so that a prognosis of the subject can be improved.

Fourth modification

In a case of repeating Steps S3 to S5 multiple times, the medical data processing device 150 may display a table indicating a correspondence relation among various parameters, accuracy in the evaluation index, and acceptance/rejection determination in each piece of the processing at Steps S3 to S5. Thus, such a modification is described as a fourth modification. In the description about the fourth modification, differences from the embodiments described above are mainly described, and the description about the same configuration as that in the embodiments described above may be omitted.

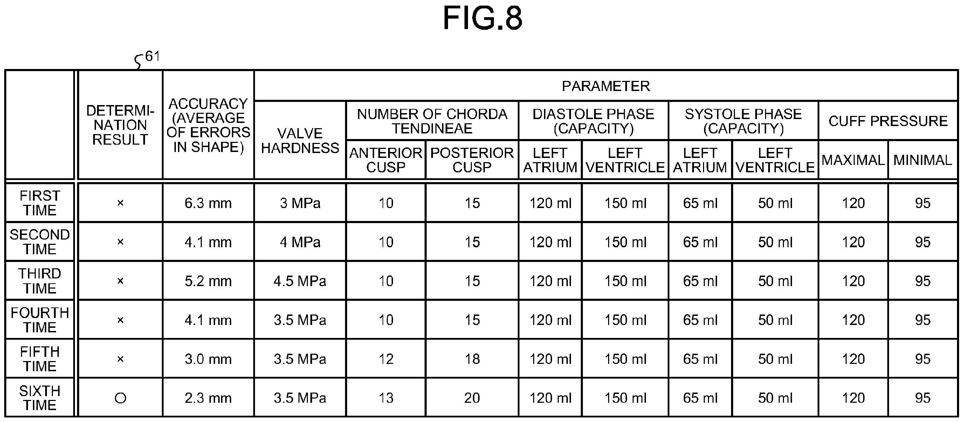

FIG. 8 is a diagram illustrating an example of a table 61 according to the fourth modification. In the fourth modification, the display control function 155e controls the display 154 to display the table 61 in FIG. 8. The table 61 includes items of "determination result", "accuracy", and "parameter" for each time (the first time to the sixth time).

If the evaluation value calculated in accordance with the expression (1) described above satisfies the reference value at Step S5 in each time (Yes at S5), a symbol "○" is registered in the item of "determination result". If the evaluation value calculated in accordance with the expression (1) described above does not satisfy the reference value at Step S5 in each time (No at S5), a symbol "X" is registered in the item of "determination result". The table 61 indicates that the evaluation value does not satisfy the reference value from the first time to the fifth time, and the evaluation value satisfies the reference value at the sixth time.

In the item of "accuracy", registered is the evaluation value that is calculated in accordance with the expression (1) described above at Step S5 in each time. The evaluation value in this example is an error in the overall shape between the estimated shape and the actually measured shape. As the evaluation value becomes smaller, the error becomes smaller.

For example, it can be found that, in a case in which the reference value is "2.5" mm, the evaluation value is larger than the reference value and does not satisfy the reference value from the first time to the fifth time, but at the sixth time, the evaluation value is "2.3" mm, which is smaller than the reference value. In this way, the table 61 indicates evaluation values of the first time to the sixth time. Due to this, the user can grasp clinically useful information such as the evaluation value not satisfying the reference value, or the evaluation value satisfying the reference value.

The item of "parameter" includes items of "valve hardness", "number of chorda tendineae", "diastole phase (capacity)", "systole phase (capacity)", and "cuff pressure".

In the item of "valve hardness", a parameter indicating hardness of the mitral valve is registered. For example, the table 61 indicates that, when the hardness of the mitral valve is increased by "1" MPa at the second time from the hardness "3" MPa of the mitral valve at the first time to set the hardness of the mitral valve to be "4" MPa, the evaluation value is reduced to be "4.1" mm from "6.3" mm. The table 61 also indicates that, when the hardness of the mitral valve is increased by "0.5" MPa at the third time from the hardness "4" MPa of the mitral valve at the second time to set the hardness of the mitral valve to be "4.5" MPa, the evaluation value is increased to be "5.2" mm, and the evaluation value is separated from the reference value. The table 61 also indicates that, even when the hardness of the mitral valve is reduced by "1" MPa at the fourth time from the hardness "4.5" MPa of the mitral valve at the third time to set the hardness of the mitral valve to be "3.5" MPa, the evaluation value at the fourth time is "4.1" mm, which is the same as the evaluation value at the second time. Thus, it can be considered that, even when the parameter indicating the hardness of the mitral valve is set to be equal to or larger than "4" MPa, the evaluation value does not come closer to the reference value, and the evaluation value becomes worse. When the hardness of the mitral valve is set to be "3.5" MPa at the fourth time, which is in a range equal to or smaller than "4" MPa and equal to or larger than "3" MPa, the evaluation value at the fourth time is the same as the evaluation value at the second time, so that it can be considered that the parameter indicating the hardness of the valve is optimized to some extent. Thus, the fourth time and subsequent processes, the medical data processing device 150 does not change the hardness of the mitral valve from "3.5" MPa. The fourth time and subsequent processes, the medical data processing device 150 changes parameters other than the parameter indicating the hardness of the mitral valve. Specifically, the fourth time and subsequent processes, the medical data processing device 150 changes the parameter related to the number of chorda tendineae.

The item of "number of chorda tendineae" includes items of "anterior cusp" and "posterior cusp". In the item of "anterior cusp", a parameter indicating the number of chorda tendineae connected to the anterior cusp is registered. In the item of "posterior cusp", a parameter indicating the number of chorda tendineae connected to the posterior cusp is registered. The table 61 indicates that the number "10" of chorda tendineae connected to the anterior cusp at the fourth time is increased by "2" at the fifth time, and the number of chorda tendineae connected to the anterior cusp is set to be "12". The table 61 indicates that the number "15" of chorda tendineae connected to the posterior cusp at the fourth time is increased by "3" at the fifth time, and the number of chorda tendineae connected to the posterior cusp is set to be "18".

By increasing the number of chorda tendineae as described above, the evaluation value is reduced to be "3.0" mm from "4.1" mm. The parameters other than the parameter related to the number of chorda tendineae are not changed from the fourth time to the fifth time. Thus, it can be considered that the parameter indicating the number of chorda tendineae connected to the anterior cusp and the parameter indicating the number of chorda tendineae connected to the posterior cusp are parameters effective for causing the evaluation value to come closer to the reference value. It can also be considered that, when the number of chorda tendineae increases, the evaluation value is reduced to come closer to the reference value. Thus, the medical data processing device 150 increases the number "12" of chorda tendineae connected to the anterior cusp at the fifth time by "1" at the sixth time, and sets the number of chorda tendineae connected to the anterior cusp to be "13". The medical data processing device 150 also increases the number "18" of chorda tendineae connected to the posterior cusp at the fifth time by "2" at the sixth time, and sets the number of chorda tendineae connected to the posterior cusp to be "20". Due to this, the evaluation value at the sixth time becomes "2.3" mm, which satisfies the reference value.

The item of "diastole phase (capacity)" includes items of "left atrium" and "left ventricle". In the item of "left atrium" included in the item of "diastole phase (capacity)", a parameter indicating the capacity of the left atrium in the diastole phase is registered. In the item of "left ventricle" included in the item of "diastole phase (capacity)", a parameter indicating the capacity of the left ventricle in the diastole phase is registered. For example, in the example of FIG. 8, registered are a parameter indicating that the capacity of the left atrium in the diastole phase is "120" (ml), and a parameter indicating that the capacity of the left ventricle in the diastole phase is "150" (ml).

The item of "systole phase (capacity)" includes items of "left atrium" and "left ventricle". In the item of "left atrium" included in the item of "systole phase (capacity)", a parameter indicating the capacity of the left atrium in the systole phase is registered. In the item of "left ventricle" included in the item of "systole phase (capacity)", a parameter indicating the capacity of the left ventricle in the systole phase is registered. For example, in the example of FIG. 8, registered are a parameter indicating that the capacity of the left atrium in the systole phase is "65" (ml), and a parameter indicating that the capacity of the left ventricle in the systole phase is "50" (ml).

The item of "cuff pressure" includes items of "maximal" and "minimal". In the item of "maximal", a parameter indicating a maximal blood pressure as the cuff pressure is registered. In the item of "minimal", a parameter indicating a minimal blood pressure as the cuff pressure is registered. For example, in the example of FIG. 8, registered are a parameter indicating that the maximal blood pressure as the cuff pressure is "120" (mmHg), and a parameter indicating that the minimal blood pressure as the cuff pressure is "95" (mmHg) .

In this way, the various parameters are included in the table 61 displayed on the display 154, so that the user can grasp clinically useful information such as the various parameters.

The table 61 indicates that, throughout the processes from the first time to the sixth time, the parameter indicating the capacity of the left atrium in the diastole phase, the parameter indicating the capacity of the left ventricle in the diastole phase, the parameter indicating the capacity of the left atrium in the systole phase, the parameter indicating the capacity of the left ventricle in the systole phase, the parameter indicating the maximal blood pressure as the cuff pressure, and the parameter indicating the minimal blood pressure as the cuff pressure are not changed.

The following describes an example of a reason why these parameters are not changed. For example, as described above, the parameter indicating the capacity of the left atrium in the diastole phase, the parameter indicating the capacity of the left ventricle in the diastole phase, the parameter indicating the capacity of the left atrium in the systole phase, and the parameter indicating the capacity of the left ventricle in the systole phase are parameters that are calculated by performing image processing on the CT image. As described above, the parameter indicating the maximal blood pressure as the cuff pressure and the parameter indicating the minimal blood pressure as the cuff pressure are parameters acquired from the electronic medical chart system. Thus, it can be considered that the six parameters including the parameter indicating the capacity of the left atrium in the diastole phase, the parameter indicating the capacity of the left ventricle in the diastole phase, the parameter indicating the capacity of the left atrium in the systole phase, the parameter indicating the capacity of the left ventricle in the systole phase, the parameter indicating the maximal blood pressure as the cuff pressure, and the parameter indicating the minimal blood pressure as the cuff pressure are relatively accurate parameters. Thus, the medical data processing device 150 does not change the six parameters described above as much as possible. The medical data processing device 150 changes the six parameters described above in a case in which the evaluation value does not satisfy the reference value even when the other parameters are changed a certain number of times. Thus, in the example of the table 61, the six parameters described above are not changed.

The display control function 155e may also control the display 154 to display part of the items instead of displaying all of the items included in the table 61. The display control function 155e may switch between causing the display 154 to display the table 61 and causing the display 154 not to display the table 61 based on an instruction from the user input via the input interface 153. The display control function 155e may also control the display 154 to display the shape of the mitral valve estimated at Step S4 in each time. The display control function 155e may also control the display 154 to display the calculation grids (meshes) set to the shape of the mitral valve at Step S4 in each time. Due to this, the user can grasp useful information including the shape of the mitral valve and the shape of the calculation grids. The display control function 155e may also control the NW interface 151 to transmit the table 61 as a log file to an external device via the network 160. Due to this, the NW interface 151 transmits the table 61 to the external device as a log file. The display control function 155e may also control the NW interface 151 to transmit, instead of the table 61 itself, information in which a plurality of items included in the table 61 are associated with each other to the external device as a log file.

As described above, in the fourth modification, the display control function 155e controls the display 154 to display the parameters used for estimating the respective estimated shapes, and the evaluation values based on the respective estimated shapes and the actually measured shape. Due to this, as described above, the user can grasp clinically useful information.

Fifth modification

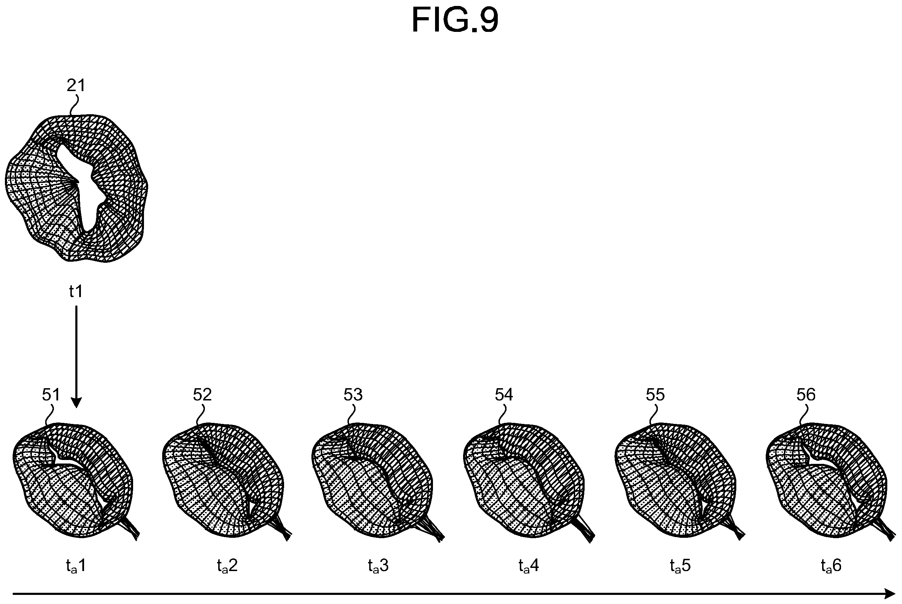

At Step S6 in each of the embodiments described above, described is a case in which the medical data processing device 150 estimates a change in the shape of the mitral valve caused by the treatment for one time point, and applies the parameter related to movement determined at Step S5 to the estimated shape of the mitral valve after the treatment at the one time point to estimate the shape of the mitral valve at another time point. FIG. 9 is a diagram for explaining an example of processing at Step S6 according to the first embodiment. As illustrated in FIG. 9, the medical data processing device 150 estimates a change in the shape of the mitral valve caused by the treatment for the one time point $t_a1$. The medical data processing device 150 then applies the parameter related to movement determined at Step S5 to the estimated shape 51 of the mitral valve after the treatment at the one time point $t_a1$ to estimate the shapes 52 to 56 of the mitral valve at the other time points $t_a2$ to $t_a6$.

However, at Step S6, the medical data processing device 150 may apply the mathematical model or the physical model to the respective calculation grids in the target region at all of the time points specified at Step S2 to estimate the shape of the mitral valve after the treatment at each of the time points. For example, the mathematical model or the physical model is a model that is set based on the parameter related to the clip and the parameter related to the shape determined at Step S5. The mathematical model or the physical model may also be a model that is set based on the parameter related to the clip and the parameter related to movement determined at Step S5. The mathematical model or the physical model may also be a model that is set based on the parameter related to the clip, and the parameter related to the shape and the parameter related to movement that are determined at Step S5. At Step S6, the medical data processing device 150 may apply the mathematical model or the physical model described above to the calculation grids in at least two or more target regions of the target regions at all of the time points specified at Step S2, and estimate the shape of the mitral valve after the treatment at each time point. Such a modification is described as a fifth modification. In the description about the fifth modification, differences from the embodiments described above are mainly described, and the description about the same configuration as that in the embodiments described above may be omitted.

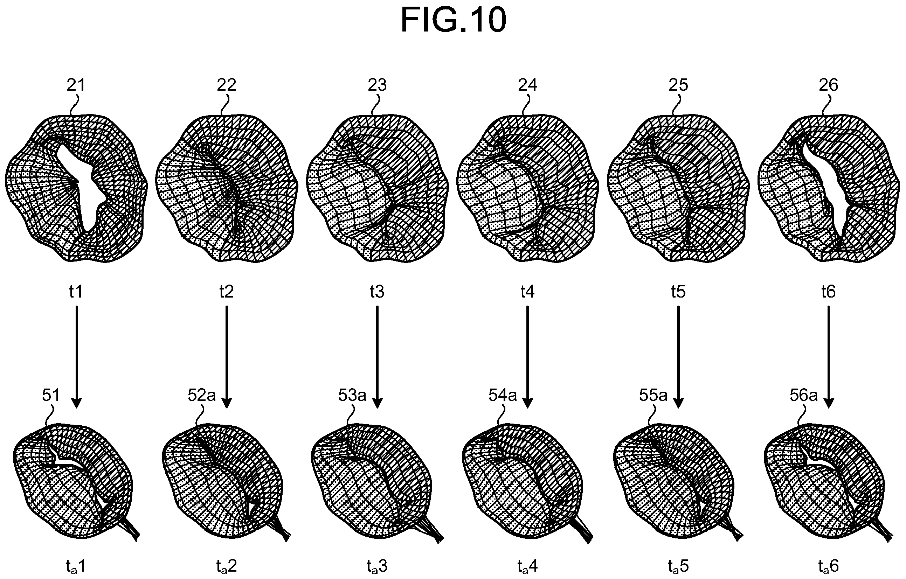

FIG. 10 is a diagram for explaining an example of processing at Step S6 according to the fifth modification. As illustrated in FIG. 10, the estimation function 155d applies the mathematical model or the physical model described above to the calculation grids set to the respective regions 21 to 26 of the mitral valve at all of the time points t1 to t6 specified at Step S2, and estimates the shapes 51 and 52a to 56a of the mitral valve after the treatment. The respective shapes 51 and 52a to 56a of the mitral valve after the treatment are shapes of the mitral valve at the respective time points $t_a1$ to $t_a6$. In this way, the estimation function 155d estimates the state of the mitral valve at the time points $t_a1$ to $t_a6$ at the timing after the treatment based on the parameter determined at Step S5 and the estimated shapes at the time points t1 to t6. The time points t1 to t6 and the time points $t_a1$ to $t_a6$ are examples of a plurality of predetermined time phases. The display control function 155e then controls the display 154 to display the estimated shapes 51 and 52a to 56a of the mitral valve after the treatment.

The estimation function 155d may apply the mathematical model or the physical model described above to the calculation grids set to respective regions of the mitral valve at at least two time points among all of the time points t1 to t6 specified at Step S2, and estimate the shape of the mitral valve after the treatment. For example, the following describes a case in which processing targets are the regions 22 and 23 of the mitral valve at the time points t2 and t3. In this case, the estimation function 155d applies the mathematical model or the physical model described above to the calculation grids set to the respective regions 22 and 23 of the mitral valve, and estimates the shapes 52a and 53a of the mitral valve after the treatment. In this way, the estimation function 155d estimates the state of the mitral valve at the timing after the treatment over a plurality of time phases based on the parameter determined at Step S5. The display control function 155e then controls the display 154 to display the estimated shapes 52a and 53a of the mitral valve after the treatment.

As described above, the medical data processing device 150 according to the fifth modification sets more appropriate parameter corresponding to respective subjects. Accordingly, similarly to the first embodiment, the medical data processing device 150 can more appropriately estimate the state of the subject after the treatment from the information about the state of the subject before the treatment.

Sixth modification

The medical data processing device 150 may perform processing of further estimating blood flow information (fluid information) after the treatment by using the form information of the mitral valve after the treatment estimated at Step S6 in each of the embodiments described above. Such a modification is described as a sixth modification. In the description about the sixth modification, differences from the embodiments described above are mainly described, and the description about the same configuration as that in the embodiments described above may be omitted.

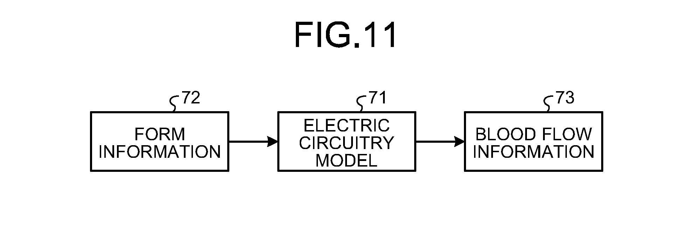

FIG. 11 is a diagram for explaining an example of processing performed by the estimation function 155d according to the sixth modification. For example, in the sixth modification, an electric circuitry model 71 illustrated in FIG. 11 is stored in the storage circuitry 152 in advance. The electric circuitry model 71 is a model simulating hemodynamics of a living body (for example, a human body). For example, the electric circuitry model 71 regards a living body as electric circuitry, regards blood as an electric current, and regards a heart as a capacitor. The estimation function 155d acquires blood flow information 73 indicating a blood flow state after the treatment by inputting, to the electric circuitry model 71, form information 72 of the mitral valve after the treatment estimated at Step S6. More specifically, the electric circuitry model 71 is designed to be able to estimate a backward flow amount of the blood flow in the mitral valve based on a valvular area of the mitral valve. The estimation function 155d then estimates the backward flow amount of the blood flow in the mitral valve after the treatment by inputting, to the electric circuitry model 71, the valvular area of the mitral valve after the treatment calculated at Step S6. The electric circuitry model 71 can be constructed based on a known Windkessel model or pulse wave propagation model.

As described above, the estimation function 155d estimates the blood flow information 73 related to the state of the blood flow (fluid) at a timing after the treatment based on the state of the form of the mitral valve estimated at Step S6 and the electric circuitry model 71 related to the hemodynamics of the living body set in advance. The blood flow information 73 is an example of state data related to a state of the fluid, and is also an example of state data indicating a state of the backward flow amount of the blood flow.

At Step S6, the estimation function 155d acquires the form information of the mitral valve by estimating the form information of the mitral valve at a timing after the treatment. The timing after the treatment is also an example of a specific timing. The form information of the mitral valve is an example of state data related to the state of the form of the biological organ. As described above, the estimation function 155d that performs operation of acquiring the form information of the mitral valve at Step S6 is also an example of an acquisition unit.

The estimation function 155d may also calculate the blood flow information without using the electric circuitry model 71. For example, the estimation function 155d may formulate required equations such as Navier-Stokes equation, the equation of continuity, Maxwell's equations, and the equation of state simultaneously, and input various parameters to the equations to calculate and obtain, as a numerical value, the blood flow information as a target.

Seventh modification

In the embodiments described above, described is a case in which the medical data processing device 150 sets the mitral valve as the target organ, but the target organ is not limited thereto. Thus, the following describes, as a seventh modification, a modification in which the medical data processing device 150 sets a biological organ other than the mitral valve to be the target organ. In the description about the seventh modification, differences from the embodiments described above are mainly described, and the description about the same configuration as that in the embodiments described above may be omitted.

For example, the medical data processing device 150 may set a coronary artery as the target organ. The medical data processing device 150 may simulate a wall shearing stress on the coronary artery. For example, at Step S1, the first acquisition function 155a acquires medical images at a plurality of time points in the diastole phase and the systole phase. At Step S2, the first acquisition function 155a acquires information about a diameter and a shape of the blood vessel in the diastole phase from the medical images. At Step S3, the calculation function 155b sets parameters such as hardness of a blood vessel wall and a blood flow speed. At Step S4, the first acquisition function 155a estimates information about the diameter and the shape of the blood vessel in the diastole phase by using the parameters set at Step S3. At Steps S3 to S5, the first acquisition function 155a and the calculation function 155b adjust the parameters so that the information about the diameter and the shape of the blood vessel in the diastole phase acquired at Step S2 becomes similar to the information about the diameter and the shape of the blood vessel in the diastole phase estimated at Step S4. At Step S6, the estimation function 155d then calculates the wall shearing stress in the systole phase using the adjusted parameters.

As described above, in the seventh modification, the first acquisition function 155a acquires the estimated data related to the state of the coronary artery in the diastole phase and the actually measured data indicating the state of the coronary artery in the diastole phase. The calculation function 155b then calculates the parameter based on the estimated data and the actually measured data. The estimation function 155d then estimates the state of the coronary artery in the systole phase based on the parameter. The diastole phase is an example of the first timing. The systole phase is an example of the second timing.

In the seventh modification, the first acquisition function 155a acquires the estimated data related to the state of the form of the coronary artery and the actually measured data indicating the state of the form of the coronary artery. The estimation function 155d then estimates the wall shearing stress on the coronary artery in the systole phase.

In another example, the medical data processing device 150 may perform a simulation of treatment related to a stimulus conduction system of a heart instead of the posttreatment simulation described above using a method similar to the posttreatment simulation described above. As a treatment method for arrhythmia, ablation treatment is known. The medical data processing device 150 sets the parameters so that anomaly in the heart before the treatment for arrhythmia matches movement. Specifically, at Step S2, the first acquisition function 155a acquires the form information of the heart before the treatment from the medical image. At Step S3, the calculation function 155b sets the parameter related to the heart. At Step S4, the first acquisition function 155a estimates the form information of the heart before the treatment using the parameter set at Step S3. At Steps S3 to S5, the first acquisition function 155a and the calculation function 155b adjust the parameters so that the form information of the heart before the treatment acquired at Step S2 becomes similar to the form information of the heart before the treatment estimated at Step S4. At Step S6, the estimation function 155d performs the posttreatment simulation for estimating movement of the heart or an estimated electric path of the stimulus conduction system after burning off biomedical tissues at respective positions of the heart by ablation treatment using the adjusted parameters.

As described above, in another example of the seventh modification, the first acquisition function 155a acquires the estimated data related to the state of the heart at the timing before the treatment and the actually measured data indicating the state of the heart at the timing before the treatment. The calculation function 155b then calculates the parameter based on the estimated data and the actually measured data. The second acquisition function 155c acquires state data related to the state of the heart in a predetermined time phase at the timing after the treatment. The estimation function 155d estimates the state of the heart at the timing after the treatment over a plurality of time phases based on the state data and the calculated parameter.

In another example of the seventh modification, the first acquisition function 155a acquires, as the state of the heart, the estimated data related to the state of the form of the heart and the actually measured data indicating the state of the form of the heart. The second acquisition function 155c then acquires the state data related to the state of the form of the heart. The estimation function 155d estimates the state of the form of the heart or the electric path of the stimulus conduction system of the heart at a timing after the treatment over a plurality of time phases.

In yet another example, the medical data processing device 150 may perform a simulation for estimating a respiratory volume of respiratory organs. For example, the medical data processing device 150 sets lungs as target organs. At Step S2, the first acquisition function 155a specifies a region of the lungs from the medical image. That is, at Step S2, the first acquisition function 155a acquires form information of the lungs before surgery from the medical image. At Step S3, the calculation function 155b sets parameters related to the lungs and a trachea. At Step S4, the first acquisition function 155a estimates movement of the lungs due to respiratory movement before the surgery using the parameters set at Step S3, and estimates the form information of the lungs at each of a plurality of time points. At Steps S3 to S5, the first acquisition function 155a and the calculation function 155b adjust the parameters so that the form information of the lungs before the surgery acquired at Step S2 becomes similar to the form information of the lungs before the surgery estimated at Step S4. At Step S6, the estimation function 155d performs a post-surgery simulation for estimating the respiratory volume of the lungs after the surgery using the adjusted parameters.

As described above, in yet another example of the seventh modification, the first acquisition function 155a acquires the estimated data related to the state of the lungs at a timing before the surgery, and the actually measured data indicating the state of the lungs at the timing before the surgery. The calculation function 155b then calculates parameters based on the estimated data and the actually measured data. The second acquisition function 155c acquires state data related to the state of the lungs in a predetermined time phase at the timing after the surgery. The estimation function 155d estimates the state of the lungs at the timing after the surgery over a plurality of time phases based on the state data and the calculated parameters. The timing before the surgery is an example of the first timing. The timing after the surgery is an example of the second timing.

In yet another example of the seventh modification, the first acquisition function 155a acquires, as the state of the lungs, estimated data related to the state of the form of the lungs and the actually measured data indicating the state of the form of the lungs. The second acquisition function 155c then acquires state data related to the state of the form of the lungs. The estimation function 155d estimates the respiratory volume of the lungs at the timing after the surgery over a plurality of time phases.

A word of "processor" used in the above description about the first embodiment means, for example, a Central Processing Unit (CPU), a Graphics Processing Unit (GPU), or circuitry such as an Application Specific Integrated Circuit (ASIC) and a programmable logic device (for example, a Simple Programmable Logic Device (SPLD), a Complex Programmable Logic Device (CPLD), and a Field Programmable Gate Array (FPGA)). Instead of storing a computer program in the storage circuitry 152, the computer program may be directly embedded in the circuitry of the processor. In this case, the processor reads out and executes the computer program embedded in the circuitry to implement the function. Each of the processors in the respective embodiments is not necessarily configured as a single piece of circuitry, but a plurality of independent pieces of circuitry may be combined and configured as one processor to implement the function.

The computer program executed by the processor is previously embedded and provided in a Read Only Memory (ROM), storage circuitry, and the like. The computer program may be recorded and provided in a computer-readable non-transitory storage medium such as a Compact Disk (CD)-ROM, a Flexible Disk (FD), a CD-Recordable (R), and a Digital Versatile Disk (DVD), as an installable or executable file. The computer program may be stored in a computer connected to a network such as the Internet, and provided or distributed by being downloaded via the network. For example, the computer program is constituted of modules including the respective processing functions described above. As actual hardware, the modules are loaded onto a main storage device and generated on the main storage device when a CPU reads out, from a storage medium such as a ROM, and executes the computer program.

According to at least one of the embodiments described above, the state of the subject at a timing such as after the treatment can be more appropriately estimated from the information related to the state of the subject at a timing such as before the treatment.

While certain embodiments have been described, these embodiments have been presented by way of example only, and are not intended to limit the scope of the inventions. Indeed, the novel embodiments described herein may be embodied in a variety of other forms; furthermore, various omissions, substitutions and changes in the form of the embodiments described herein may be made without departing from the scope of the invention as defined by the appended claims.

**Claims**

1. A medical data processing device comprising:

   a first acquisition unit (155a) configured to acquire estimated data related to a state of a biological organ at a first timing, and measured data indicating the state of the biological organ at the first timing,
   a calculation unit (155b) configured to calculate a parameter based on the estimated data and the measured data, and
   an estimation unit (155d) configured to estimate a state of the biological organ in a predetermined time phase at a second timing different from the first timing based on the parameter and the estimated data in the predetermined time phase.

2. The medical data processing device according to claim 1, wherein

   the calculation unit (155b) is configured to calculate, as the parameter, a parameter related to a shape of a first biological organ based on the estimated data and the measured data, and
   the estimation unit (155d) is configured to estimate the state of the biological organ in the predetermined time phase at the second timing based on the parameter related to the shape of the first biological organ and the estimated data in the predetermined time phase.

3. The medical data processing device according to claim 2, wherein

   the calculation unit (155b) is configured to calculate, as the parameter related to the shape, a parameter indicating at least one of hardness, a thickness, a fiber direction, a length, a width, a connecting position, and number of the first biological organ based on the estimated data and the measured data, and
   the estimation unit (155d) is configured to estimate the state of the biological organ in the predetermined time phase at the second timing based on the parameter indicating at least one of the above items and the estimated data in the predetermined time phase.

4. The medical data processing device according to any one of claims 1 to 3, wherein the calculation unit (155b) is configured to calculate the parameter used for estimating the estimated data in a case in which an evaluation value calculated based on the estimated data and the measured data satisfies a standard determined in advance.

5. The medical data processing device according to claim 4, wherein the calculation unit (155b) is configured to calculate the parameter used for estimating the estimated data in a case in which the estimated data is similar to the measured data.

6. The medical data processing device according to any one of claims 1 to 5, wherein

   the first acquisition unit (155a) is configured to acquire a plurality of pieces of the estimated data by estimating the pieces of estimated data using a plurality of different parameters, and
   the calculation unit (155b) is configured to calculate the parameter used for estimating the state of the biological organ based on the pieces of estimated data and the measured data.

7. The medical data processing device according to any one of claims 1 to 6, wherein

   the first acquisition unit (155a) is configured to acquire the estimated data at a timing before treatment as the first timing, and the measured data at the timing before the treatment, and
   the estimation unit (155d) is configured to estimate the state of the biological organ at a timing after the treatment as the second timing.

8. The medical data processing device according to any one of claims 1 to 7, wherein

   the first acquisition unit (155a) is configured to acquire the estimated data related to a state of a mitral valve as the biological organ, and the measured data indicating the state of the mitral valve, and
the estimation unit (155d) is configured to estimate the state of the mitral valve at the second timing.

9. The medical data processing device according to any one of claims 1 to 8, wherein

   the first acquisition unit (155a) is configured to acquire the estimated data related to a state of a form of the biological organ as the state of the biological organ, and the measured data indicating the state of the form of the biological organ, and
   the estimation unit (155d) is configured to estimate the state of the form of the biological organ at the second timing.

10. The medical data processing device according to any one of claims 1 to 9, wherein

    the first acquisition unit (155a) is configured to acquire the estimated data related to a state of a blood flow in the biological organ as the state of the biological organ, and the measured data indicating the state of the blood flow in the biological organ, and
    the estimation unit (155d) is configured to estimate the state of the blood flow in the biological organ at the second timing.

11. The medical data processing device according to any one of claims 1 to 10, wherein the first acquisition unit (155a) is configured to acquire the estimated data and the measured data using medical images of at least two time phases among medical images of three or more time phases at the first timing, the medical images in which the biological organ is delineated.

12. The medical data processing device according to any one of claims 1 to 11, wherein

    the calculation unit (155b) is configured to further change a calculation parameter for performing processing of estimating the estimated data, and
    the first acquisition unit (155a) is configured to further estimate the estimated data based on the changed calculation parameter.

13. The medical data processing device according to claim 9, wherein the estimation unit (155d) is configured to estimate state data related to a state of a fluid at the second timing based on the estimated state of the form of the biological organ and an electric circuitry model related to hemodynamics of a living body set in advance.

14. The medical data processing device according to any one of claims 1 to 13, wherein

    the first acquisition unit (155a) is configured to acquire the estimated data by performing a simulation for acquiring the estimated data, and
    the calculation unit (155b) is configured to calculate, as the parameter, a parameter for bringing the estimated data closer to the measured data and for adjusting the simulation.

15. A medical data processing method comprising:

    acquiring estimated data related to a state of a biological organ at a first timing, and measured data indicating the state of the biological organ at the first timing;
    calculating a parameter based on the estimated data and the measured data; and
    estimating a state of the biological organ in a predetermined time phase at a second timing different from the first timing based on the parameter and the estimated data in the predetermined time phase.

# FIG.1

# FIG.2

```
        ┌──────────────┐
        │    START     │
        └──────┬───────┘
               │           ⌐S1
        ┌──────▼───────────────┐
        │ ACQUIRE MEDICAL IMAGE │
        └──────┬───────────────┘
               │           ⌐S2
        ┌──────▼───────────────────┐
        │ ACQUIRE FORM INFORMATION  │
        └──────┬───────────────────┘
               │           ⌐S3
        ┌──────▼───────────────────┐
        │      SET PARAMETER        │
        └──────┬───────────────────┘
               │           ⌐S4
        ┌──────▼───────────────────┐
        │  PERFORM PRETREATMENT     │
        │       SIMULATION          │
        └──────┬───────────────────┘
               │           ⌐S5
          ◇ IS RESULT OF
   NO ← PRETREATMENT SIMULATION
          DETERMINED? ◇
               │ YES      ⌐S6
        ┌──────▼───────────────────┐
        │  POSTTREATMENT SIMULATION │
        └──────┬───────────────────┘
               │           ⌐S7
        ┌──────▼───────────────────┐
        │ DISPLAY ESTIMATION RESULT │
        └──────┬───────────────────┘
               │
        ┌──────▼───────┐
        │     END      │
        └──────────────┘
```

# FIG.3

EP 3 992 981 A1

# FIG.4

PARAMETER SETTING

32 ☑ HARDNESS OF VALVE :

35 → [ 3 ] MPa

33 ☑ NUMBER OF CHORDA TENDINEAE:

ANTERIOR CUSP [ 10 ] ← 36

POSTERIOR CUSP [ 15 ]

37

34 ☐ CONSIDER POSITION OF PAPILLARY MUSCLE

31

# FIG.5

# FIG.6

21

51

t1

t$_a$1

# FIG.7

## FIG.8

| | DETERMI-NATION RESULT | ACCURACY (AVERAGE OF ERRORS IN SHAPE) | PARAMETER | | | | | | | | |
|---|---|---|---|---|---|---|---|---|---|---|---|
| | | | VALVE HARDNESS | NUMBER OF CHORDA TENDINEAE | | DIASTOLE PHASE (CAPACITY) | | SYSTOLE PHASE (CAPACITY) | | CUFF PRESSURE | |
| | | | | ANTERIOR CUSP | POSTERIOR CUSP | LEFT ATRIUM | LEFT VENTRICLE | LEFT ATRIUM | LEFT VENTRICLE | MAXIMAL | MINIMAL |
| FIRST TIME | × | 6.3 mm | 3 MPa | 10 | 15 | 120 ml | 150 ml | 65 ml | 50 ml | 120 | 95 |
| SECOND TIME | × | 4.1 mm | 4 MPa | 10 | 15 | 120 ml | 150 ml | 65 ml | 50 ml | 120 | 95 |
| THIRD TIME | × | 5.2 mm | 4.5 MPa | 10 | 15 | 120 ml | 150 ml | 65 ml | 50 ml | 120 | 95 |
| FOURTH TIME | × | 4.1 mm | 3.5 MPa | 10 | 15 | 120 ml | 150 ml | 65 ml | 50 ml | 120 | 95 |
| FIFTH TIME | × | 3.0 mm | 3.5 MPa | 12 | 18 | 120 ml | 150 ml | 65 ml | 50 ml | 120 | 95 |
| SIXTH TIME | ○ | 2.3 mm | 3.5 MPa | 13 | 20 | 120 ml | 150 ml | 65 ml | 50 ml | 120 | 95 |

61

# FIG.9

EP 3 992 981 A1

FIG.10

# FIG.11

```
        72                    71                   73
 ┌─────────────┐      ┌─────────────┐      ┌─────────────┐
 │    FORM     │ ───▶ │  ELECTRIC   │ ───▶ │ BLOOD FLOW  │
 │ INFORMATION │      │  CIRCUITRY  │      │ INFORMATION │
 │             │      │    MODEL    │      │             │
 └─────────────┘      └─────────────┘      └─────────────┘
```

Europäisches
Patentamt

European
Patent Office

Office européen
des brevets

# EUROPEAN SEARCH REPORT

**Application Number**

EP 21 20 5526

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 9 245 091 B2 (VOIGT INGMAR [DE]; IONASEC RAZVAN IOAN [US] ET AL.) 26 January 2016 (2016-01-26) * the whole document * * in particular * * column 3 - column 7 * * column 10 - column 15 * ----- | 1-15 | INV. G16H30/40 G16H20/40 G16H50/50 G16H50/20 |
| X | US 2012/232386 A1 (MANSI TOMMASO [US] ET AL) 13 September 2012 (2012-09-13) * the whole document * * in particular * * paragraph [0027] - paragraph [0114] * ----- | 1-15 | |
| X | US 2016/171766 A1 (GRBIC SASA [US] ET AL) 16 June 2016 (2016-06-16) * the whole document * * in particular * * paragraph [0025] - paragraph [0126] * ----- | 1-15 | |

**TECHNICAL FIELDS SEARCHED (IPC)**

G16H

The present search report has been drawn up for all claims

| Place of search | Date of completion of the search | Examiner |
|---|---|---|
| Munich | 19 March 2022 | Rákossy, Z |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

........................................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 20 5526

This annex lists the patent family members relating to the patent documents cited in the above-mentioned European search report.
The members are as contained in the European Patent Office EDP file on
The European Patent Office is in no way liable for these particulars which are merely given for the purpose of information.

19-03-2022

| Patent document cited in search report | | Publication date | Patent family member(s) | Publication date |
|---|---|---|---|---|
| US 9245091 | B2 | 26-01-2016 | NONE | |
| US 2012232386 | A1 | 13-09-2012 | NONE | |
| US 2016171766 | A1 | 16-06-2016 | NONE | |

EPO FORM P0459

For more details about this annex : see Official Journal of the European Patent Office, No. 12/82